(19)

Europäisches
Patentamt
European
Patent Office
Office européen
des brevets

(11) **EP 4 002 141 A1**

(12) **EUROPEAN PATENT APPLICATION**
published in accordance with Art. 153(4) EPC

(43) Date of publication:
**25.05.2022 Bulletin 2022/21**

(21) Application number: **20900702.0**

(22) Date of filing: **29.05.2020**

(51) International Patent Classification (IPC):
***G06F 16/00*** *(2019.01)*

(52) Cooperative Patent Classification (CPC):
**G06F 16/00**

(86) International application number:
**PCT/CN2020/093390**

(87) International publication number:
**WO 2021/237707 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BOE Technology Group Co., Ltd.**
**Beijing 100015 (CN)**

(72) Inventors:
• WANG, Yu
  **Beijing 100176 (CN)**

• **HE, Wangqiang**
  **Beijing 100176 (CN)**
• **WANG, Hong**
  **Beijing 100176 (CN)**
• **WANG, Yufeng**
  **Beijing 100176 (CN)**
• **LEI, Yiming**
  **Beijing 100176 (CN)**

(74) Representative: **Brötz, Helmut et al**
**Rieder & Partner mbB**
**Patentanwälte - Rechtsanwalt**
**Yale-Allee 26**
**42329 Wuppertal (DE)**

(54) **QUESTION RECOMMENDATION METHOD, APPARATUS AND SYSTEM, AND ELECTRONIC DEVICE AND READABLE STORAGE MEDIUM**

(57)     A question recommendation method, a device, a system, an electronic device, and a non-volatile readable storage medium are provided. The question recommendation method includes: obtaining a candidate question set of a user, the candidate question set including a plurality of candidate questions; obtaining user behavior data, and obtaining a user interest parameter based on the user behavior data; based on the user interest parameter and the plurality of candidate questions, obtaining at least one similarity feature between each candidate question of the plurality of candidate questions and the user interest parameter; based on basic user information, the plurality of candidate questions, and the at least one similarity feature, sorting the plurality of candidate questions to obtain a question sequence; and based on an order of the question sequence, recommending at least one candidate question in the question sequence to the user.

10

FIG. 1A

**Description**

TECHNICAL FIELD

Embodiments of the present disclosure relate to a question recommendation method, a question recommendation device, a question recommendation system, an electronic device, and a non-volatile readable storage medium.

BACKGROUND

With rapid development of Internet technology, a lot of convenience may be provided to people's life. People can search and browse information of interest through the Internet, and can also conduct online consultation, online diagnosis, or the like through the Internet. The Internet has accumulated a large amount of content information over time, search results on the traditional search engine web page are large in quantity, and there may be much repetitive and irrelevant content, which is difficult for the user to find information of interest, the question that the user wants to consult, or the like in a short time.

SUMMARY

At least one embodiment of the present disclosure provides a question recommendation method, and the question recommendation method comprises: obtaining a candidate question set of a user, the candidate question set comprising a plurality of candidate questions; obtaining user behavior data, and obtaining a user interest parameter based on the user behavior data; based on the user interest parameter and the plurality of candidate questions, obtaining at least one similarity feature between each candidate question of the plurality of candidate questions and the user interest parameter; based on basic user information, the plurality of candidate questions, and the at least one similarity feature, sorting the plurality of candidate questions to obtain a question sequence; and based on an order of the question sequence, recommending at least one candidate question in the question sequence to the user.

For example, in the question recommendation method provided by at least one embodiment of the present disclosure, based on the basic user information, the plurality of candidate questions, and the at least one similarity feature, sorting the plurality of candidate questions to obtain the question sequence, comprises: by using a ranking model, combining the basic user information, the plurality of candidate questions, and the at least one similarity feature to form an input feature vector of the ranking model, obtaining a score corresponding to each candidate question of the plurality of candidate questions, and sorting the plurality of candidate questions according to a value of the score corresponding to each candidate question of the plurality of candidate questions, so as to obtain the question sequence.

For example, in the question recommendation method provided by at least one embodiment of the present disclosure, based on the user interest parameter and the plurality of candidate questions, obtaining at least one similarity feature between each candidate question of the plurality of candidate questions and the user interest parameter, comprises: by using at least one similarity matching model, obtaining the at least one similarity feature between each candidate question and the user interest parameter based on the user interest parameter and the plurality of candidate questions.

For example, in the question recommendation method provided by at least one embodiment of the present disclosure, the at least one similarity matching model comprises at least one of a group consisting of a cosine similarity model, a Jaccard similarity model, an edit distance similarity model, a word mover's distance similarity model, and a deep structured semantic similarity model.

For example, in the question recommendation method provided by at least one embodiment of the present disclosure, the ranking model comprises a Wide&Deep model.

For example, in the question recommendation method provided by at least one embodiment of the present disclosure, obtaining the candidate question set of the user comprises: accessing a data knowledge base, the data knowledge base comprising a plurality of knowledge question sets; obtaining the basic user information, and establishing a user tag set based on the basic user information; and associating the user tag set with the data knowledge base, and obtaining the candidate question set according to the plurality of knowledge question sets.

For example, in the question recommendation method provided by at least one embodiment of the present disclosure, the user tag set comprises a multi-level tag set, the multi-level tag set comprises tags of a plurality of levels, and tags of different levels are of different types.

For example, in the question recommendation method provided by at least one embodiment of the present disclosure, the question recommendation method is used to recommend a question related to disease, a first-level tag of the multi-level tag set is an age group, a second-level tag of the multi-level tag set is a time period, a third-level tag of the multi-level tag set is a type of disease, and a fourth-level tag of the multi-level tag set is a complication.

For example, in the question recommendation method provided by at least one embodiment of the present disclosure, each of the plurality of knowledge question sets comprises: a standard question, a standard answer corresponding to the standard question, and an extended question corresponding to the standard question.

For example, in the question recommendation method provided by at least one embodiment of the present disclosure, obtaining the candidate question set of the user further comprises: establishing the data knowledge base.

For example, in the question recommendation method provided by at least one embodiment of the present disclosure, establishing the data knowledge base comprises: retrieving a data set from a network, and classifying the data set according to intention to form the plurality of knowledge question sets, so as to establish the data knowledge base.

For example, in the question recommendation method provided by at least one embodiment of the present disclosure, the question recommendation method is used to recommend a question related to disease, and the data set is based on at least one of a group consisting of a medical consultation data set between a doctor and a patient, a hotspot question associated with the disease, and a reward question associated with the disease.

For example, in the question recommendation method provided by at least one embodiment of the present disclosure, associating the user tag set with the data knowledge base, and obtaining the candidate question set according to the plurality of knowledge question sets, comprises: establishing a mapping relationship between the user tag set and the standard question in the data knowledge base, matching the user tag set with the standard question in the data knowledge base, and combining the knowledge question set corresponding to a matched standard question to form the candidate question set.

For example, in the question recommendation method provided by at least one embodiment of the present disclosure, obtaining the candidate question set of the user comprises: retrieving the candidate question set of the user stored beforehand.

For example, in the question recommendation method provided by at least one embodiment of the present disclosure, obtaining the user interest parameter based on the user behavior data comprises: analyzing the user behavior data, and converting a question clicked by the user or a word or sentence which the user is interested in into the user interest parameter.

At least one embodiment of the present disclosure provides a question recommendation device, and the question recommendation device comprises: a set acquisition circuit, configured to obtain a candidate question set of a user, the candidate question set comprising a plurality of candidate questions; a behavior analysis circuit, configured to obtain user behavior data and obtain a user interest parameter based on the user behavior data; a feature generation circuit, configured to obtain at least one similarity feature between each candidate question of the plurality of candidate questions and the user interest parameter based on the user interest parameter and the plurality of candidate questions; a question sorting circuit, configured to sort the plurality of candidate questions to obtain a question sequence based on basic user information, the plurality of candidate questions, and the at least one similarity feature; and a recommendation circuit, configured to recommend at least one candidate question in the question sequence to the user based on an order of the question sequence.

For example, in the question recommendation device provided by at least one embodiment of the present disclosure, the question sorting circuit comprises: a question sorting sub-circuit, configured to, by using a ranking model, combine the basic user information, the plurality of candidate questions, and the at least one similarity feature to form an input feature vector of the ranking model, obtain a score corresponding to each candidate question of the plurality of candidate questions, and sort the plurality of candidate questions according to a value of the score corresponding to each candidate question of the plurality of candidate questions, so as to obtain the question sequence.

For example, in the question recommendation device provided by at least one embodiment of the present disclosure, the set acquisition circuit comprises: a knowledge base access circuit, configured to access a data knowledge base, the data knowledge base comprising a plurality of knowledge question sets; an information acquisition circuit, configured to obtain the basic user information and establish a user tag set based on the basic user information; and a candidate set generation circuit, configured to associate the user tag set with the data knowledge base and obtain the candidate question set according to the plurality of knowledge question sets, the candidate question set comprising the plurality of candidate questions.

For example, in the question recommendation device provided by at least one embodiment of the present disclosure, the set acquisition circuit further comprises: a knowledge base establishing circuit, configured to retrieve a data set from a network and classify the data set according to intention to form the plurality of knowledge question sets, so as to establish the data knowledge base.

For example, in the question recommendation device provided by at least one embodiment of the present disclosure, the candidate set generation circuit comprises: a candidate set generation sub-circuit, configured to establish a mapping relationship between the user tag set and the standard question in the data knowledge base, match the user tag set with the standard question in the data knowledge base, and combine the knowledge question set corresponding to a matched standard question to form the candidate question set.

For example, in the question recommendation device provided by at least one embodiment of the present disclosure, the behavior analysis circuit comprises: a behavior analysis sub-circuit, configured to analyze the user behavior data and convert a question clicked by the user or a word or sentence which the user is interested in into the user interest parameter.

For example, in the question recommendation device provided by at least one embodiment of the present disclosure, the feature generation circuit comprises: a feature generation sub-circuit, configured to, by using at least one similarity matching model, obtain the at least one similarity feature between each candidate question and the user interest parameter based on the user interest parameter and the plurality of candidate questions.

At least one embodiment of the present disclosure provides a question recommendation system, and the question recommendation system comprises a terminal and a question recommendation server. The terminal is configured to send request data to the question recommendation server. The question recommendation server is configured to, in response to the request data, obtain a candidate question set of a user which comprises a plurality of candidate questions, obtain user behavior data, and obtain a user interest parameter based on the user behavior data, based on the user interest parameter and the plurality of candidate questions, obtain at least one similarity feature between each candidate question of the plurality of candidate questions and the user interest parameter, and based on basic user information, the plurality of candidate questions, and the at least one similarity feature, sort the plurality of candidate questions to obtain a question sequence. The terminal is further configured to display first N candidate questions in the question sequence, and N is an integer greater than or equal to 1.

At least one embodiment of the present disclosure provides an electronic device. The electronic device comprises: a processor; and a memory, comprising one or more computer program modules. The one or more computer program modules are stored in the memory and configured to be executed by the processor, and the one or more computer program modules comprise instructions for performing the question recommendation method according to any one of the embodiments of the present disclosure.

At least one embodiment of the present disclosure provides a non-volatile readable storage medium, where computer instructions are stored. The question recommendation method according to any one of the embodiments of the present disclosure is executed in the case where the computer instructions are executed by a processor.

BRIEF DESCRIPTION OF THE DRAWINGS

In order to clearly illustrate the technical solutions of the embodiments of the present disclosure, the drawings of the embodiments will be briefly described in the following. It is obvious that the described drawings are only related to some embodiments of the present disclosure and thus are not limitative to the present disclosure.

FIG. 1A is an exemplary flowchart of a question recommendation method provided by at least one embodiment of the present disclosure;

FIG. 1B is an exemplary flowchart of another question recommendation method provided by at least one embodiment of the present disclosure;

FIG. 2A illustrates a user interface of a certain platform according to at least one embodiment of the present disclosure;

FIG. 2B illustrates a schematic diagram of establishing a user tag set according to at least one embodiment of the present disclosure;

FIG. 3A illustrates a schematic diagram of a rule scheme between a user tag set and a data knowledge base according to at least one embodiment of the present disclosure;

FIG. 3B illustrates another user interface of the certain platform according to at least one embodiment of the present disclosure;

FIG. 4A is a schematic structural diagram of a Wide&Deep model provided by at least one embodiment of the present disclosure;

FIG. 4B illustrates still another user interface of the certain platform according to at least one embodiment of the present disclosure;

FIG. 4C illustrates further still another user interface of the certain platform according to at least one embodiment of the present disclosure;

FIG. 5A is an exemplary flowchart of another question recommendation method provided by at least one embodiment of the present disclosure;

FIG. 5B is a schematic block diagram of the question recommendation method in FIG. 5A provided by at least one embodiment of the present disclosure;

FIG. 6 is a schematic block diagram of a question recommendation device provided by at least one embodiment of the present disclosure;

FIG. 7 is a schematic block diagram of a question recommendation system provided by at least one embodiment of the present disclosure;

FIG. 8 is a schematic block diagram of an electronic device provided by at least one embodiment of the present disclosure;

FIG. 9 is a schematic block diagram of a terminal provided by at least one embodiment of the present disclosure;

FIG. 10 is a schematic block diagram of a non-volatile readable storage medium provided by at least one embodiment of the present disclosure; and

FIG. 11 illustrates an exemplary scene diagram of a question recommendation system provided by at least one embodiment of the present disclosure.

DETAILED DESCRIPTION

In order to make objects, technical details and advantages of the embodiments of the present disclosure apparent, the technical solutions of the embodiments will be described in a clearly and fully understandable way in connection with the drawings related to the embodiments of the present disclosure. Apparently, the described embodiments are just a part but not all of the embodiments of the present disclosure. Based on the described embodiments herein, those skilled in the art can obtain other embodiment(s), without any inventive work, which should be within the scope of the present disclosure.

Unless otherwise defined, all the technical and scientific terms used herein have the same meanings as commonly understood by one of ordinary skill in the art to which the present disclosure belongs. The terms "first," "second," etc., which are used in the description and the claims of the present application for disclosure, are not intended to indicate any sequence, amount or importance, but distinguish various components. Also, the terms such as "a," "an," etc., are not intended to limit the amount, but indicate the existence of at least one. The terms "comprise," "comprising," "include," "including," etc., are intended to specify that the elements or the objects stated before these terms encompass the elements or the objects and equivalents thereof listed after these terms, but do not preclude the other elements or objects. The phrases "connect," "connected," "coupled," etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

With economic development and improvement of living standards, more and more people suffer from chronic diseases of varying degrees. The chronic disease refers to the general term for diseases which do not constitute infection and have long-term accumulation of disease form damage. Common chronic diseases mainly include cardiovascular and cerebrovascular diseases, cancer, diabetes, chronic respiratory diseases, etc. Cardiovascular and cerebrovascular diseases include hypertension, cerebral stroke, coronary heart disease, etc. Statistics show that one of the causes of chronic diseases is an unhealthy lifestyle. For example, the unhealthy lifestyle includes irrational diet, insufficient exercise, tobacco use, excessive alcohol use, etc. Therefore, for patients with chronic diseases, in addition to medical treatment (for example, treatment with drugs) for patients according to conditions, doctors also need to provide patients with reasonable advice (for example, dietary advice, exercise advice, etc.) on coping with chronic diseases as the auxiliary means of medical treatment, so as to better control and prevent chronic diseases. With development of network technology, smart medical care provides more convenience, for example, especially for health knowledge related to chronic disease prevention, control and self-inspection, which can improve patients' awareness of diseases and conduct more effective prevention and treatment. Therefore, the intelligent question answering system for health education provided for patients with chronic diseases can efficiently and accurately solve doubts and questions of patients. At the same time, under the current domestic shortage of medical resources, the intelligent question answering system for health education can also reduce the burden on medical workers and facilitate long-term health management.

The inventors of the present disclosure have noticed that, currently, the medical question answering system or online consultation relies on the condition described by the patient and the question raised by the patient. Usually, because the information expressed by the patient is not clear enough, the feedback answer provided by the system may be inadequate for patient information or mismatched in sequence. In addition, general medical question answering systems do not use user information to provide targeted health knowledge, which is difficult for providing personalized and diversified service.

At least one embodiment of the present disclosure provides a question recommendation method, a question recommendation device, a question recommendation system, an electronic device, and a non-volatile readable storage medium. The question recommendation method includes: obtaining a candidate question set of a user, the candidate question set including a plurality of candidate questions; obtaining user behavior data, and obtaining a user interest parameter based on the user behavior data; based on the user interest parameter and the plurality of candidate questions, obtaining at least one similarity feature between each candidate question of the plurality of candidate questions and the user interest parameter; based on basic user information, the plurality of candidate questions, and the at least one similarity feature, sorting the plurality of candidate questions to obtain a question sequence; and based on an order of the question sequence, recommending at least one candidate question in the question sequence to the user.

The question recommendation method provided by at least one embodiment of the present disclosure not only effectively avoids the question of inappropriate feedback answers due to unclear expression of the patient, but also can provide personalized question recommendation regarding to individual factors, self-characteristics, and the like (for example, basic user information, click behaviors and browsing behaviors of the user, etc.), so that the users can more pertinently learn the knowledge related to their own health. In at least some other embodiments, the question recommendation method can also make the question recommendation more relevant, personalized, and diversified by using a ranking model, and further pay attention to the final feedback order, thereby achieving the effect of better satisfaction of requirements of the user and effectively improving user experience.

The following is a non-limiting description of the question recommendation method provided by at least one embodiment of the present disclosure through several examples or embodiments. As described below, different features in these specific examples or embodiments can be combined with each other to obtain new examples or embodiments in case of no conflict, and these new examples or embodiments also fall within the protection scope of the present disclosure.

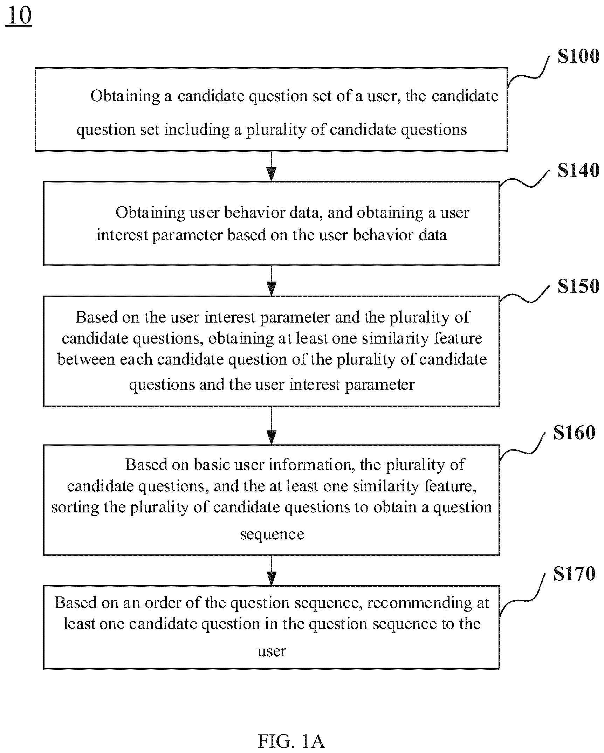

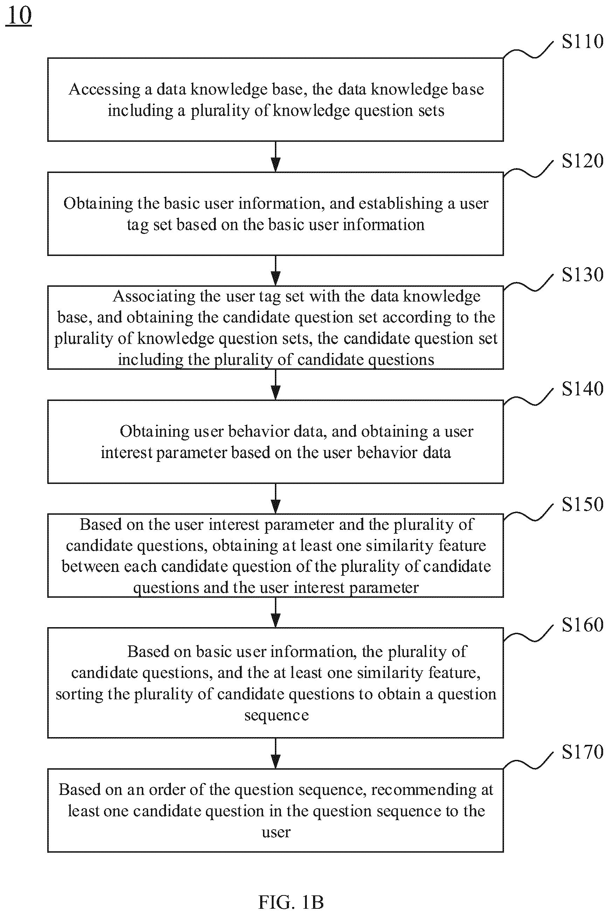

FIG. 1A is an exemplary flowchart of a question recommendation method provided by at least one embodiment of the present disclosure, and FIG. 1B is an exemplary flowchart of another question recommendation method provided by at least one embodiment of the present disclosure.

A question recommendation method 10 provided by at least one embodiment of the present disclosure may be applied to scenarios such as medical intelligent question answering, online diagnosis, health consultation, etc., and for example, can be applied to an intelligent question answering system of chronic disease health knowledge. For example, in an embodiment, as illustrated in FIG. 1A, the question recommendation method 10 may include the following operations.

Step S100: obtaining a candidate question set of a user, the candidate question set including a plurality of candidate questions.

Step S140: obtaining user behavior data, and obtaining a user interest parameter based on the user behavior data.

Step S150: based on the user interest parameter and the plurality of candidate questions, obtaining at least one similarity feature between each candidate question of the plurality of candidate questions and the user interest parameter.

Step S160: based on basic user information, the plurality of candidate questions, and the at least one similarity feature, sorting the plurality of candidate questions to obtain a question sequence.

Step S170: based on an order of the question sequence, recommending at least one candidate question in the question sequence to the user.

For example, in an embodiment, as illustrated in FIG. 1B, step S100 in the question recommendation method 10 may specifically include step S110 to step S130, and thus the question recommendation method 10 may specifically include step S110 to step S170.

The question recommendation method 10 provided by the embodiments of the present disclosure will be described in detail below with reference to FIG. 1A and FIG. 1B. For example, in an embodiment of the present disclosure, the question recommendation method 10 may specifically include step S110 to step S170 as illustrated in FIG. 1B. For example, step S110 to step S170 may be executed sequentially or executed in other adjusted order. For example, in an example, step S110 may be executed first and then step S120 may be executed; and in another example, step S120 may be executed first and then step S110 may be executed. For another example, part or all of the operations in step S110 to step S160 may also be executed in parallel. For example, step S110 and step S120 may be executed in parallel. The embodiments of the present disclosure do not limit the execution sequence of respective steps, which may be adjusted according to actual conditions. For example, step S110 to step S160 can be implemented by a server or a local terminal, which is not limited in the embodiments of the present disclosure. For example, in some examples, implementing the question recommendation method 10 provided by at least one embodiment of the present disclosure may selectively perform some steps in step S110 to step S170, or may perform some additional steps in addition to step S110 to step S170, and the embodiments of the present disclosure are not specifically limited in this aspect.

The question recommendation method 10 provided by the present disclosure will be described in detail below with reference to the accompanying drawings through examples.

In step S110, a data knowledge base is accessed, and the data knowledge base includes a plurality of knowledge question sets. For example, in the case where the question recommendation method 10 is applied to an intelligent question answering system for chronic disease health knowledge, the data knowledge base can be a data knowledge base associated with chronic diseases (for example, diabetes, hypertension, etc.), and includes complete basic health knowledge related to chronic diseases. The data knowledge base includes a plurality of knowledge question sets. For example, the basic health knowledge in the data knowledge base is classified according to intention (such as diet, exercise, medication, examination, complications, surgery, treatment, symptoms, etc.), and standard questions and corresponding extended questions and standard answers are sorted (for example, manually sorted) under each intention to form one knowledge question set, thus forming the data knowledge base.

For example, in at least one embodiment of the present disclosure, each of the plurality of knowledge question sets may include the standard question, the standard answer corresponding to the standard question, and the extended question corresponding to the standard question. For example, in an example, the user is a diabetic patient. Under the intention of "diet," the sorted standard question may include "What foods should the diabetic patient eat?", and the extended question corresponding to the standard question may include, for example, "What foods are good for the diabetic patient?", "What foods are suitable for the diabetic patient?", "What foods should the diabetic patient eat to lower the level of blood glucose?", etc. In this case, the knowledge question set under the intention of above-mentioned "diet" may include the aforementioned standard question and extended questions, and corresponding standard answers.

It should be noted that the standard question and extended question listed in the embodiments of the present disclosure are only illustrative, and can be adjusted and updated according to application scenarios, medical practices, etc. In addition, similarly, the standard answer can also be adjusted and updated according to application scenarios, medical practices, etc., and can further be adjusted and updated according to the adopted language (for example, Chinese, English, etc.), which is not specifically limited in the embodiments of the present disclosure.

For example, in the embodiments of the present disclosure, the data knowledge base may be already established, and the established data knowledge base may be pre-stored in the local terminal or in the server, or may be established such as by the server when implementing the question recommendation method 10, and the data knowledge base may also be read from other devices. The embodiments of the present disclosure are not specifically limited in this aspect, which can be set according to actual needs. Detailed description of establishing the data knowledge base will be described in the following.

Step S120: obtaining the basic user information, and establishing a user tag set based on the basic user information.

For example, in at least one embodiment of the present disclosure, the basic user information may include, for example, the age, gender, height, weight, waist circumference, lifestyle habits, and the like of the user. For example, in an example, if the user is a diabetic patient, the basic user information also includes the type of diabetes, confirmed chronic complications, existing symptoms, and the like. For example, in another example, if the user is not a diabetic patient, the basic user information also includes the medical history of the user, fasting blood glucose level, two-hour postprandial blood glucose level, or the like. For example, in still another example, if the user is a hypertensive patient, the basic user information also includes the diastolic blood pressure, systolic blood pressure, type of hypertension comorbidity, symptoms, and the like of the user. The embodiments of the present disclosure do not specifically limit the content included in the basic user information, which can be set according to actual needs.

For example, in an example, the basic user information may be obtained from the online real-time information of the user, for example, from an online health record platform, an information management system (for example, a laboratory information management system) of a medical institution (for example, a hospital or a medical examination institution), an electronic device for physical examination report, and the like under permission.

For example, in an example, in the case where the user is a registered user of a certain software platform (for example, a health management platform), the user voluntarily fills in and saves the basic information (for example, the name, gender, age, height, weight, waist circumference, lifestyle habits, etc.) while registering, and thus the basic information of the user can be obtained directly from the database (for example, back stage) associated with the specific platform. For example, in another example, in the case where the user is not a registered user of the specific platform (e.g., the health management platform) or the user has not completed or saved the basic information in the specific platform, the basic information of the user may be collected by a third-party platform (for example, an information management system of a hospital or a medical examination institution, etc.) or related electronic devices (for example, a smart bracelet, a smart watch, etc.). The embodiments of the present disclosure are not specifically limited in this aspect, which can be adjusted according to actual conditions. For example, in an example, when a user uses a certain webpage or a certain platform for the first time, the basic information of the user may also be obtained according to the personal information entered by the user in the text box. The embodiments of the present disclosure are not specifically limited in this aspect, which can be adjusted according to actual conditions.

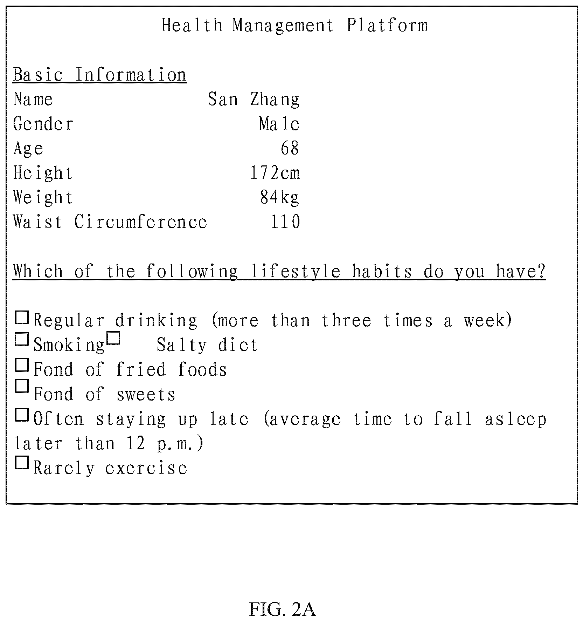

FIG. 2A illustrates a user interface of a certain platform according to at least one embodiment of the present disclosure. As illustrated in FIG. 2A, users can fill in basic information according to self-conditions (for example, including name, gender, age, height, weight, waist circumference, and the like), and then check the lifestyle habits according to the specific lifestyle options (for example, including regular drinking (more than three times a week), smoking, salty diet, fond of fried foods, fond of sweets, often staying up late (average time to fall asleep later than 12 p.m.), rarely exercise, etc.) provided on the user interface. The basic information of the user may be saved in a database (for example, back stage) associated with the health management platform.

For example, the health management platform may interface with at least one (for example, multiple) medical institution, and obtain basic information and examination results of patients participating in physical examination at the medical institution. For example, the health management platform may also obtain basic user information from smart terminals (for example, smart measuring instruments, smart bracelets, smart watches, smart clothes, etc.) as well as at least one physical sign data (for example, the pulse, body temperature, heart rate, respiration, brain electricity, electrocardiogram, blood pressure, blood glucose, myoelectricity, etc.) of the patient detected by the sensor in the smart terminal.

For example, the health management platform may periodically (for example, daily) obtain the examination results of patients participating in physical examination at these medical institutions from multiple medical institutions, and store the examination results in a database (or a memory) associated with the health management platform in advance. It should be noted that the embodiments of the present disclosure do not specifically limit the source of the basic user information, and the source can be set according to actual needs. For example, in some other examples, the basic user information can also be filled in by the user online. For example, a corresponding web page may be provided, the user fills in self information in the web page, the web page sends the information filled by the user to the server, and the server organizes the information to obtain the basic user information.

For example, in an example, after the basic user information is obtained according to the above content, a tag set is established based on the obtained basic user information.

For example, the tag of the user may include gender, name, age, and the like of the user. For example, in an example, the tag also includes the name of the disease, such as type I diabetes, etc. For example, in another example, the tag also includes the name of the complication, such as diabetic foot, etc.

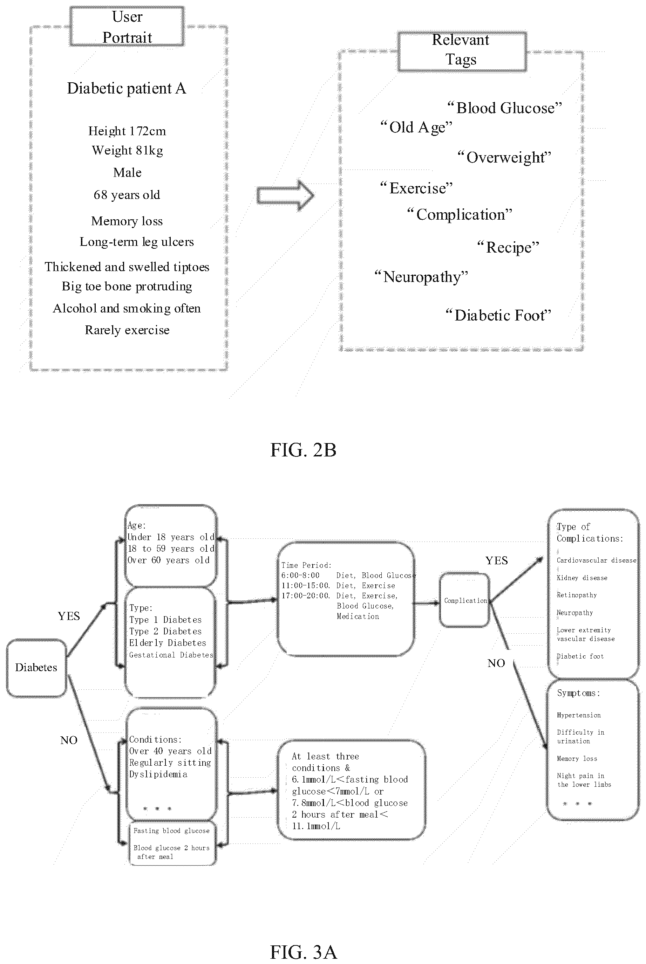

The process of establishing the tag set based on the obtained basic user information is described below in conjunction with FIG. 2B. FIG. 2B illustrates a schematic diagram of establishing a user tag set according to at least one embodiment of the present disclosure. As illustrated in FIG. 2B, the dashed block on the left is the user portrait, that is, the basic user information. The basic user information includes: "diabetic patient A," "height 172cm," "weight 81kg," "male," "68 years old," "memory loss," "long-term leg ulcers," "thickened and swelled tiptoes," "big toe bone protruding," "alcohol and smoking often," and "rarely exercise." Then, for example, the tag is obtained through methods such as entity recognition and the like. The entity recognition method includes, for example, using methods such as conditional random field, deep learning, and the like to obtain the tag. For example, the dashed block on the right is the obtained related tag of the user, that is, the user tag set includes: "blood glucose," "old age," "overweight," "complication," "recipe," "neuropathy," and "diabetic foot."

It should be noted that the specific tags listed in the description of the foregoing embodiments of the present disclosure are only illustrative and not limitative.

For example, in at least one embodiment of the present disclosure, the user tag set may include a multi-level tag set, the multi-level tag set includes tags of a plurality of levels, and tags of different levels are of different types.

For example, in at least one embodiment of the present disclosure, nearly two hundred basic rules can be established according to different age groups, time periods, types of disease, presence or absence of symptoms, presence or absence of complications, etc., which may spread to different types of diabetes, combination of multiple types of symptoms, and combination of multiple types of complications. Thus, the multi-level tag set can cover the basic information and interest orientation of the user.

For example, in an example, the first-level tag of the multi-level tag set is an age group, the second-level tag of the multi-level tag set is a time period, the third-level tag of the multi-level tag set is a type of disease, and the fourth-level tag of the multi-level tag set is a complication.

It should be noted that the embodiments of the present disclosure do not limit the specific rules of the user tag set, which can be set according to actual needs.

Step S130: associating the user tag set with the data knowledge base, and obtaining the candidate question set according to the plurality of knowledge question sets, the candidate question set including the plurality of candidate questions.

For example, in at least one embodiment of the present disclosure, the user tag set established in step S120 can be matched with the data knowledge base accessed in step S110, and the candidate question set can be obtained according to rule association.

For example, in at least one embodiment of the present disclosure, in step S130, associating the user tag set with the data knowledge base and obtaining the candidate question set according to the plurality of knowledge question sets includes: first establishing a mapping relationship between the user tag set and the standard question in the data knowledge base, matching the user tag set with the standard question in the data knowledge base, and then combining the knowledge question set corresponding to the matched standard question to form the candidate question set.

FIG. 3A illustrates a schematic diagram of a rule scheme between a user tag set and a data knowledge base according to at least one embodiment of the present disclosure. The operation in step S130 is described in detail below with reference to FIG. 3A by taking the basic rule scheme of diabetes as an example.

As illustrated in FIG. 3A, in at least one embodiment of the present disclosure, the age group is used as the first-level tag to first directly associate with the user (for example, a diabetic patient) in a rule association manner. For example, when the user is associated with the first-level tag, the user may be associated with three categories, i.e., minors under 18 years old, newborns, and children, adults within the range of 18 years old to 59 years old, and adults over 60 years old.

For different age groups, different corresponding rule schemes are provided combining the second-level tag (that is, different time periods of the day) and the third-level tag (e.g., diabetes types).

For example, there is no limitation on the order of the specific implementation of tags of respective levels. For example, the age group, time period, and type of diabetes are a single choice, which may be more convenient to classify first, and the type of complications and the like may be with multiple choice, which is more complicated and determined later.

For example, in at least one embodiment of the present disclosure, for children with type 1 and type 2 diabetes, during the breakfast period (for example, 6:00-8:00), questions related to diet and nutrition advice, notes for blood glucose 2 hours after breakfast, and the like may be consulted. For example, in an example, for children with type 1 and type 2 diabetes, during the breakfast period (for example, 6:00-8:00), the corresponding tag includes "type 1 and type 2 diabetes," "child," and "6:00-8:00," and the corresponding candidate questions may include: "What should children with diabetes eat for breakfast?", "What are the precautions for children with type 1 and type 2 diabetes after breakfast?", "What equipment does the diabetic patient use to measure the blood glucose level," etc. For children with type 1 and type 2 diabetes, during the lunch period (for example, 11:00-15:00), questions related to diet and nutrition advice, exercise advice, precautions, and the like may be consulted. For example, in an example, for children with type 1 and type 2 diabetes, during the lunch period (for example, 11:00-15:00), the corresponding tag includes "type 1 and type 2 diabetes," "child," and "11:00-15:00," and the corresponding candidate questions may include: "What should children with diabetes eat for lunch?", "What are the precautions for children with type 1 and type 2 diabetes after lunch?", "What exercises are suitable for children with type 1 and type 2 diabetes after lunch?", etc. For children with type 1 and type 2 diabetes, during the dinner period (for example, 17:00-20:00), questions related to diet and nutrition advice, exercise advice, precautions, medication-related notice, and the like may be consulted. For example, in an example, for children with type 1 and type 2 diabetes, during the dinner period (for example, 17:00-20:00), the corresponding tag includes "type 1 and type 2 diabetes," "child," and "17:00-20:00," and the corresponding candidate questions may include: "What should children with type 1 and type 2 diabetes eat for dinner?", "What are the precautions for diabetic patients after dinner?", "What exercises are suitable for children with diabetes after dinner?", "How do children with type 1 and type 2 diabetes take medicine at night?", etc. It should be noted that the recommendation questions listed in the embodiments of the present disclosure are only exemplary and not limitative.

For example, in at least one embodiment of the present disclosure, for adults with type 1 and type 2 diabetes, during the breakfast period (for example, 6:00-8:00), questions related to diet and nutrition advice, notes for blood glucose 2 hours after breakfast, and the like may be consulted. For example, in an example, for adults with type 1 and type 2 diabetes, during the breakfast period (for example, 6:00-8:00), the corresponding tag includes "type 1 and type 2 diabetes," "adult," and "6:00-8:00," and the corresponding candidate questions may include: "What should adults with diabetes eat for breakfast?", "What are the precautions for adults with type 1 and type 2 diabetes after breakfast?", "Can adult diabetic patients drink milk in the morning?", etc. During the lunch period (for example, 11:00-15:00), questions related to diet and nutrition advice, exercise advice, precautions, and the like may be consulted. For example, in an example, for adults with type 1 and type 2 diabetes, during the lunch period (for example, 11:00-15:00), the corresponding tag includes "type 1 and type 2 diabetes," "adult," and "11:00-15:00," and the corresponding candidate questions may include: "What should adults with type 1 and type 2 diabetes eat for lunch?", "What are the precautions for adults with diabetes after lunch?", "What exercises are suitable for adults with type 1 and type 2 diabetes after lunch?", "How do adults with type 1 and type 2 diabetes take medicine at noon?", etc. During the dinner period (for example, 17:00-20:00), questions related to diet and nutrition advice, exercise advice, precautions, notes for blood glucose 2 hours after dinner, medication-related notice, and the like may be consulted. For example, in an example, for adults with type 1 and type 2 diabetes, during the dinner period (for example, 17:00-20:00), the corresponding tag includes "type 1 and type 2 diabetes," "adult," and "17:00-20:00," and the corresponding candidate questions may include: "What should adults with type 1 and type 2 diabetes eat for dinner?", "What are the precautions for adults with type 1 and type 2 diabetes after dinner?", "What exercises are suitable for adults with type 1 and type 2 diabetes after dinner?", "How do adults with type 1 and type 2 diabetes take medicine at night?", etc. It should be noted that the recommendation questions listed in the embodiments of the present disclosure are only exemplary and not limitative.

For example, in at least one embodiment of the present disclosure, for elderly diabetic patients and gestational diabetic patients, during the breakfast period (for example, 6:00-8:00), questions related to diet and nutrition advice, notes for blood glucose 2 hours after breakfast, and the like may be consulted. For example, in an example, for elderly diabetic patients and gestational diabetic patients, during the breakfast period (for example, 6:00-8:00), the corresponding tag includes "diabetes," "gestational diabetes," "elder," and "6:00-8:00," and the corresponding candidate questions may include: "What should elderly diabetic patients and gestational diabetic patients eat for breakfast?", "What are the precautions for elderly diabetic patients and gestational diabetic patients after breakfast?", "How do elderly diabetic patients and gestational diabetic patients take medicine in the morning?", etc. During the lunch period (for example, 11:00-15:00), questions related to diet and nutrition advice, notes for blood glucose 2 hours after lunch, exercise advice, precautions, and the like may be consulted. For example, in an example, for elderly diabetic patients and gestational diabetic patients, during the lunch period (for example, 11:00-15:00), the corresponding tag includes "diabetes," "gestational diabetes," "elder," and "11:00-15:00," and the corresponding candidate questions may include: "What should elderly diabetic patients and gestational diabetic patients eat for lunch?", "What are the precautions for elderly diabetic patients and gestational diabetic patients after lunch?", "What exercises are suitable for elderly diabetic patients and gestational diabetic patients after lunch?", "How do elderly diabetic patients and gestational diabetic patients take medicine at noon?", etc. During the dinner period (for example, 17:00-20:00), questions related to diet and nutrition advice, exercise advice, precautions, notes for blood glucose 2 hours after dinner, medication-related notice, and the like may be consulted. For example, in an example, for elderly diabetic patients and gestational diabetic patients, during the dinner period (for example, 17:00-20:00), the corresponding tag includes "diabetes," "gestational diabetes," "elder," and "17:00-20:00," and the corresponding candidate questions may include: "What should elderly diabetic patients and gestational diabetic patients eat for dinner?", "What are the precautions for elderly diabetic patients and gestational diabetic patients after dinner?", "What exercises are suitable for elderly diabetic patients and gestational diabetic patients after dinner?", "How do elderly diabetic patients and gestational diabetic patients take medicine at night?", etc. It should be noted that the recommendation questions listed in the embodiments of the present disclosure are only exemplary and not limitative.

For example, in at least one embodiment of the present disclosure, for pre-diabetes patients, during the breakfast period (for example, 6:00-8:00), questions related to diet and nutrition advice, notes for blood glucose 2 hours after breakfast, and the like may be consulted. For example, in an example, for pre-diabetes patients, during the breakfast period (for example, 6:00-8:00), the corresponding candidate questions may include: "What should pre-diabetes patients eat for breakfast?", "What are the precautions for pre-diabetes patients after breakfast?", "How do pre-diabetes patients take medicine in the morning?", etc. During the lunch period (for example, 11:00-15:00), questions related to diet and nutrition advice, exercise advice, precautions, and the like may be consulted. For example, in an example, for pre-diabetes patients, during the lunch period (for example, 11:00-15:00), the corresponding candidate questions may include: "What should pre-diabetes patients eat for lunch?", "What are the precautions for pre-diabetes patients after lunch?", "How do pre-diabetes patients take medicine at noon?", etc. During the dinner period (for example, 17:00-20:00), questions related to diet and nutrition advice, exercise advice, precautions, and the like may be consulted. For example, in an example, for pre-diabetes patients, during the dinner period (for example, 17:00-20:00), the corresponding candidate questions may include: "What should pre-diabetes patients eat for dinner?", "What are the precautions for pre-diabetes patients after dinner?", "What exercises are suitable for pre-diabetes patients after dinner?", "How do pre-diabetes patients take medicine at night?", etc. It should be noted that the recommendation questions listed in the embodiments of the present disclosure are only exemplary and not limitative.

For example, in at least one embodiment of the present disclosure, after the first-level tag (for example, the age group), the second-level tag (for example, the time period of a day), and the third-level tag (for example, the type of disease) are determined, the complication may serve as the fourth-level tag for rule association. According to clinical data, about 10 years after the onset of diabetes, 30% to 40% of patients may have at least one complication, such as cardiovascular disease, kidney disease, retinopathy, neuropathy, lower extremity vascular disease, diabetic foot, etc.

For example, in some exemplary rule schemes, if there is no confirmed complication, questions related to one or more complications are selected and recommended according to the existing symptoms of the user, and then these related questions are sent to the candidate question set corresponding to the user, that is, to form the plurality of candidate questions corresponding to the user. For example, in the case of no confirmed complication, the existing symptoms of the user are combined with clinical data so as to recommend questions related to one or more complications. For example, in an example, the high blood pressure, pain in the precordial area, palpitation, chest tightness, and the like are symptoms of cardiovascular disease complications. For example, when the user already has symptoms of the high blood pressure and pain in the precordial area, the user tag set may include the tag "cardiovascular disease," which can recommend some questions related to cardiovascular disease complications, for example, "What are the symptoms of cardiovascular disease complications?", "Why do you feel palpitation and chest tightness?", "What should you do if you have palpitation and chest tightness?", etc. The embodiments of the present disclosure are not specifically limited in this aspect. For example, in another example, foamy urine, difficulty in urination, lower extremity edema, eyelid edema, and the like are symptoms of complications of diabetic nephropathy. For example, in another example, blurred vision, decreased vision, vision blurring, and the like are complications of retinopathy. For example, in another example, slurred speech, decreased memory, persistent numbness and tingling of hands and feet, swelling, and the like are neuropathic complications. For example, in another example, intermittent numbness and weakness of the lower limbs, claudication, night pain in the lower limbs, and the like are complications of lower limb vascular disease. For example, in another example, long-term leg ulcers, thickened and swelled fingertips or tiptoes, big toe bone protruding, and the like are symptoms of diabetic foot complications.

For example, if a complication has been diagnosed, related questions can be recommended based on the complication. For example, in an example, if the user tag includes "cardiovascular disease," the matched questions in the data knowledge base may include: "If the diabetic patient has cardiovascular disease complications, what treatment should be used?", "What are the symptoms of cardiovascular disease complications of the diabetic patient?", etc. The embodiments of the present disclosure are not specifically limited in this aspect.

It should be noted that the classification of various diseases and the classification of various symptoms in complications described in the embodiments of the present disclosure are only to describe how to establish the mapping relationship between the user tag set and the data knowledge base, that is, only to illustrate the above specific rule schemes for purpose of description. The classification and symptom analysis of specific diseases may be adjusted and set based on a large amount of clinical data, professional experience judgments, etc. The embodiments of the present disclosure are not specifically limited in this aspect.

With reference to FIG. 3A, it can be seen that the above descriptions are all situations where the user chooses the selection of diabetes, and if the user chooses the selection of no diabetes, the following operations can be performed. For example, if the user meets at least three of the following conditions: regularly sitting, first-degree relatives with a history of diabetes, high blood pressure, dyslipidemia, a history of impaired glucose regulation, a history of giving birth to a large baby, a woman with a history of gestational diabetes, a patient with atherosclerotic cardiovascular and cerebrovascular diseases, etc., and also has one of the following conditions: 6. 1mmol/L< fasting blood glucose<7mmol/L, and 7.8mmol/L<blood glucose 2 hours after meal<11 .1mmol/L, the data knowledge base is matched according to the pre-diabetes rules to form the candidate question set.

For example, in at least one embodiment of the present disclosure, the user is a hypertensive patient. The rule scheme for hypertensive patients is different from the rule scheme for diabetic patients. For example, in an example, the rule scheme of a hypertensive patient can be directly determined as pre-hypertension, mild hypertension, moderate hypertension, and severe hypertension according to values of diastolic blood pressure and systolic blood pressure, so as to execute different rule schemes. For example, in the rule scheme for hypertensive patients, for the first-level tag (i.e., the age group), a stage is specifically set for the elderly over 80 years old, which is classified as critical elderly hypertension, and blood pressure must be monitored at any time. In addition, because diabetes is a common complication of hypertension, it is also necessary to simultaneously monitor blood pressure and blood glucose at a specific time in the rule scheme for hypertensive patients.

It should be noted that the rule scheme provided by the embodiments of the present disclosure is only illustrative, and the embodiments of the present disclosure are not limited in the specific rule scheme, which may be set according to actual needs.

For example, in the embodiments of the present disclosure, after the mapping relationship between the user tag set and the standard question in the data knowledge base is established, the user tag set is matched with the standard question in the data knowledge base by using methods such as entity recognition, keyword matching, deep learning, etc. Then the knowledge question set corresponding to the matched standard question is retrieved from the data knowledge base, so as to form the candidate question set. In the above, the entity recognition refers to the recognition of entities with specific meanings in the text, for example, a name of a person, a place, time, etc. For example, the keyword matching method includes broad matching, exact matching, phrase matching, negative matching, and the like. For example, in an example, if the text content of the tag is "diabetes," it can be matched to the recommendation question containing the word "diabetes" in the data knowledge base, for example, "What is the recipe for diabetic patients?", "What exercise is suitable for diabetic patients?", "What are the symptoms of diabetic patients?", or the like, and the embodiments of the present disclosure are not specifically limited in this aspect.

For example, in an example, an elderly person over 60 years old with type 1 diabetes and complications usually wants to get the corresponding diet and advice on blood glucose monitoring, and also wants to know the questions related to complications at 12 p.m. Through the foregoing steps, a corresponding user tag set is established based on the basic information of the user (i.e., including tags: "type 1 diabetes," "complication," "over 60 years old" and "12 p.m."). The user tag set is associated with the data knowledge base, a candidate question set is obtained from multiple knowledge question sets included in the data knowledge base, and the candidate question set includes multiple candidate questions. For example, according to the user tag set, a plurality of related candidate questions can be matched from the data knowledge base by methods such as entity recognition, keyword matching, deep learning, and the like, so to form the candidate question set. For example, the plurality of candidate questions corresponding to the user in the above example may include: "What are the recipes for three meals a day for diabetic patients?", "What exercise is suitable for elderly diabetic patients?", "How often do diabetic patients monitor blood glucose every day?", "What are the symptoms of patients with type 1 diabetes?", and "How to treat diabetic complications and peripheral neuropathy?".

Therefore, by establishing the mapping relationship between the user tag set and the standard question in the data knowledge base, convenient conditions are provided for quickly retrieving the corresponding knowledge question set (including the standard question, the extended question, and the standard answer) from the data knowledge base through the user tag set while establishing rule association in the subsequent process.

Step S 140: obtaining user behavior data, and obtaining a user interest parameter based on the user behavior data.

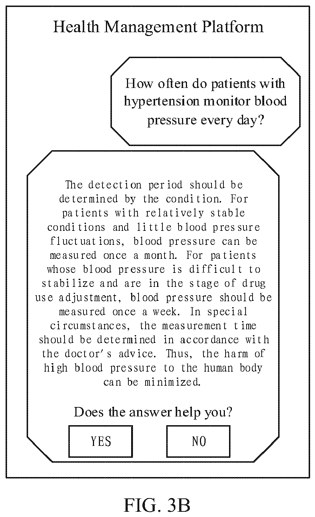

For example, in at least one embodiment of the present disclosure, user behavior data (for example, user behavior logs) can be acquired from software on a client terminal or a Web server, or user behavior data can also be collected by customization, which is not limited in the embodiments of the present disclosure. For example, the user behavior data can include all behavior data while the user visits a website, such as visiting, browsing, clicking, etc. That is, the user behavior data can feed back the specific behavior of the user, for example, which link or which page the user clicks on, which search term does the user adopt, etc. For example, in at least one embodiment of the present disclosure, the user interest parameter can be obtained by analyzing the user behavior data described above. For example, in an example, the feedback behavior of the user may include the explicit feedback behavior and the implicit feedback behavior. For example, the explicit feedback behavior includes the explicit feedback of the user on the answer, such as clearly choosing whether the answer is helpful. As illustrated in FIG. 3B, in an example, on a certain software platform (for example, a health management platform), when a user raises a certain question, for example, "How often do patients with hypertension monitor blood pressure every day?", the platform will consult the user: "Does the answer help you?" after providing the answer to the question. According to the behavior of clicking on "YES" or "NO" of the user, the feedback of the user on the answer can be clearly known, which can reflect the interest and concern of the user. The implicit feedback behavior refers to the fact that cannot directly reflect the preference of the user, but reflects the preference of the user in an indirect way, for example, through the frequency of click and browse of the user within a certain period of time. For example, in an example, the health knowledge read by the user can be summarized by the maximal marginal relevance (MMR) algorithm. The sentence in the document can be extracted according to the importance to form the summary by the MMR algorithm, the high-frequency words in the abstract can be obtained by the term frequencyinverse document frequency (TF-IDF) method, and these high-frequency words (also called keywords) are also important features reflecting the interest and concern of the user.

For example, in an example, if a user uses a specific software platform (for example, a health management platform) in a certain device for the first time, user behavior data can be obtained by analyzing the log of the application stored in the device. The log may be a log stored after startup and operation of the device this time, or may be a log stored after startup and operation of the device last time. The embodiments of the present disclosure are not specifically limited in this aspect, which can be adjusted according to actual conditions.

For example, in an example, the question clicked by the user, the high-frequency word browsed by the user, the keyword, and the like, which reflect the interest and concern of the user, are converted into the user interest parameter. For example, the user interest parameter may be a numerical vector to reflect the interest and concern of the user. For example, word embedding is performed on the question clicked by the user or the word and sentence that the user is interested in, and the embedding vector of the user-interest word and sentence is generated, which forms the aforementioned "user interest parameter." The word embedding can be understood as a mapping relationship, which can map or embed a word in a text space into another numerical vector space by a certain method. In other words, the word embedding can express the vocabulary and complete sentence in vector form.

Step S150: based on the user interest parameter and the plurality of candidate questions, obtaining at least one similarity feature between each candidate question of the plurality of candidate questions and the user interest parameter.

For example, in at least one embodiment of the present disclosure, based on the user interest parameter and the plurality of candidate questions, obtaining at least one similarity feature between each candidate question of the plurality of candidate questions and the user interest parameter may include: by using at least one similarity matching model, obtaining the at least one similarity feature between each candidate question and the user interest parameter based on the user interest parameter and the plurality of candidate questions.

For example, in at least one embodiment of the present disclosure, the at least one similarity matching model includes at least one of a group consisting of a cosine similarity model, a Jaccard similarity model, an edit distance (Levenshtein) similarity model, a word mover's distance (WMD) similarity model, and a deep structured semantic (DSSM) similarity model.

For example, in an example, a user interest parameter A (for example, a numerical vector) is provided, any candidate question (for example, a standard question or an extended question) in the candidate set is subjected to word embedding to generate the embedding vector B of the candidate question, and the vector B is also a numerical vector. The word embedding can be understood as a mapping relationship, which can map or embed a word in a text space into another numerical vector space by a certain method. In other words, the word embedding can express the vocabulary and complete sentence in vector form. The user interest parameter A and a certain candidate question B are input into the above-mentioned similarity models, and through each of the similarity models, each similarity model outputs the similarity features between the numerical vectors A and B. The larger the value of the similarity feature, the more similar the word and sentence corresponding to the vector A and the word and sentence corresponding to the vector B. The embodiments of the present disclosure do not limit the number of similarity matching models. For example, in an example, if five similarity matching models are used, there may be five similarity features between the vectors A and B. For example, in an example, if three similarity matching models are used, the vectors A and B may have three similarity features.

In the following, several similarity matching models mentioned above are briefly introduced.

(1) The cosine similarity: the cosine similarity uses the cosine value of the vector angle as a measure of the difference between two individuals. The closer the cosine value is to 1, the more similar the two vectors A and B are. The following formula is usually used to calculate the cosine similarity (also known as the cosine distance):

$$similarity = \cos(\theta) = \frac{A \cdot B}{\|A\|\|B\|} = \frac{\sum_{i=1}^{n} A_i \times B_i}{\sqrt{\sum_{i=1}^{n} A_i^2} \times \sqrt{\sum_{i=1}^{n} B_i^2}}$$

In addition, the similarity features output by the cosine similarity model are continuous.

(2) The Jaccard distance: the Jaccard distance is used to measure the degree of discrimination between two sets according to the proportion of different elements to all elements in two sets. It is expressed by the following formula, and J(A, B) is the Jaccard similarity coefficient.

$$d_j(A, B) = 1 - J(A, B) = \frac{|A \cup B| - |A \cap B|}{|A \cup B|} = \frac{A \Delta B}{|A \cup B|}$$

In addition, the similarity features output by the Jaccard similarity model are continuous.

(3) The edit distance, also known as the Levenshtein distance, refers to the minimum number of operations required to convert string A into string B by using character operations. The permitted character operations include modifying a character, inserting a character, and deleting a character. Generally speaking, the smaller the edit distance of two strings, the more similar the two strings are. If two strings are identical, the edit distance of the two strings is 0. In addition, the similarity features output by the edit distance similarity model are continuous.

(4) The word mover's distance (WMD) refers to considering the similarity between two documents as a whole, and measuring the semantic similarity of documents by searching the pair of the minimum distance between all words in the two documents. The similarity features output by the WMD similarity model are continuous.

(5) The DSSM model: the DSSM model is a deep structured semantic model, which maps the two matched to low-dimensional space, and the correlation question is transformed into the distance of low-dimensional space vectors. The model can not only be used to predict the semantic similarity of two sentences, but also can obtain the low-dimensional semantic vector expression of the sentences. In addition, the similarity features output by the DSSM model are discrete.

For example, in at least one embodiment of the present disclosure, the above five similarity matching models can be used for the user interest parameter and candidate questions at the same time, and then for each candidate question of the candidate questions, five similarity features corresponding to the five similarity matching models and regarding to the user interest parameters can be obtained.

It should be noted that the similarity matching model used in the embodiments of the present disclosure may not be limited to the similarity matching model described above, and other similarity matching models can also be used, as long as the same or similar technical effects can be achieved, that is, the similarity between two vectors can be calculated, which is not specifically limited in the embodiments of the present disclosure. In addition, the embodiments of the present disclosure do not limit the number of similarity matching models used, which can be set according to actual needs.

Step S160: based on basic user information, the plurality of candidate questions, and the at least one similarity feature, sorting the plurality of candidate questions to obtain a question sequence.

For example, in at least one implementation of the present disclosure, based on basic user information, the plurality of candidate questions, and the at least one similarity feature, sorting the plurality of candidate questions to obtain a question sequence may include: by using a ranking model, combining the basic user information, the plurality of candidate questions, and the at least one similarity feature to form an input feature vector of the ranking model, obtaining a score corresponding to each candidate question of the plurality of candidate questions, and sorting the plurality of candidate questions according to a value of the score (for example, in order of the score from high to low) corresponding to each candidate question of the plurality of candidate questions, so as to obtain the question sequence.

For example, in at least one embodiment of the present disclosure, the basic information of a certain user includes: gender is male (for example, the corresponding discrete feature is "0"), and suffering from diabetes (for example, the corresponding discrete feature is "1"). The embedding vector corresponding to one of the candidate questions is [0.3, 0.5, 0.6]. The similarity feature between the candidate question and the user interest parameter includes: cosine similarity of 0.85, Jaccard distance of 0.91, edit distance of 3, WMD of 1.17, and DSSM of 2. In this case, the input feature vector that composes them into the ranking model is a vector [0, 1, 0.3, 0.5, 0.6, 0.85, 0.91, 3, 1.17, 2]. It should be noted that the feature data provided in this embodiment is only exemplary, the specific value of the feature data may be set according to experimental results or actual conditions, and the embodiments of the present disclosure are not specifically limited in this aspect.

Here, the basic user information, the plurality of candidate questions, and the at least one similarity feature form the input feature vector of the ranking model, so that the individual factors of the user (for example, basic user information, user behavior data, etc.) are fully considered for personalized question recommendation, which allows the user to more specifically learn knowledge related to self-health.

For example, in at least one embodiment of the present disclosure, the ranking model used is the classic Wide&Deep model proposed by Google. In the above example, the vector [0, 1, 0.3, 0.5, 0.6, 0.85, 0.91, 3, 1.17, 2] is input to the Wide&Deep model as the input feature vector. The core idea of the model is to combine the memory ability of the linear model with the generalization ability of the deep neural network model, and reflect the integration of relevance and diversity in the recommended scene. This model is used to score the candidate questions in the question candidate set, and the corresponding candidate questions are sorted in the order of the scores from high to low, so as to obtain the question sequence. Then, according to actual needs, the first N questions in the question sequence are shown to the user, where N is an integer greater than or equal to 1.

For example, in at least one embodiment of the present disclosure, the score of a candidate question can be expressed as a conditional probability p(y|x). For example, y represents a tag corresponding to a certain user behavior. For example, if the user clicks on the candidate question, then y=1; and if the user does not click on the candidate question, then y=0. Among them, x represents the input feature vector. The input feature vector x includes the discrete features of the basic user information described above, the continuous embedding vector of the candidate question, and at least one similarity feature between the candidate question and the user interest parameter (the similarity feature may be of continuous or discrete type). For example, the probability value P(y=1|x) output according to y=1 is used as the final score of the candidate question, so that the score (i.e., the probability value) of each candidate question in the candidate question set can be output through the ranking model (i.e., the Wide&Deep model).

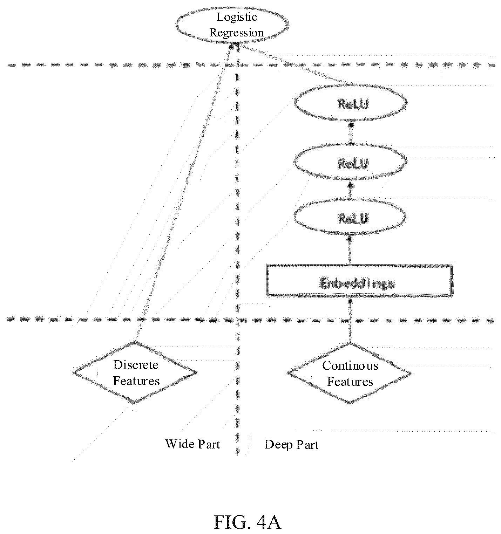

The principle of the Wide&Deep model is briefly introduced below in conjunction with FIG. 4A. FIG. 4A is a schematic structural diagram of a Wide&Deep model provided by at least one embodiment of the present disclosure.

As illustrated in FIG. 4A, the Wide&Deep model includes two parts, that is, the Wide part (on the left side of FIG. 4A) and the Deep part (on the right side of FIG. 4A). The Wide&Deep model balances the memory and generalization capabilities of the Wide model and the Deep model. The two models require different input features.

For the Wide part, the Wide model is a generalized linear model (for example, logistic regression) with the following formula:

$$y = w^T x + b$$

For example, x represents the feature vector [x1, x2, x3...], w represents the parameter vector [w1, w2, w3....], b is the bias term, and y is the output tag (label). The output is a probability value between 0 and 1 by the sigmoid function. The input features used in the Wide part are discrete features, for example, discrete features of basic user information and discrete similarity features.

For the Deep part, the Deep model is a feed-forward neural network. Generally, the input of the deep neural network model is continuous dense features. The sparse highdimensional features need to be subjected to embedding (dimensionality reduction) so as to be converted to low-dimensional dense features, which are used as the input of the first hidden layer and trained and updated in the reverse direction according to the final error (loss). The activation function f of the hidden layer usually adopts the ReLU function that prevents the gradient from disappearing. Therefore, the input features used in the Deep part are continuous features, such as the embedding vector of the candidate question and the continuous similarity feature.

During model training, the gradient is calculated according to the final error and back-propagated to the Wide part and Deep part to continuously update the parameters of the model, so as to obtain the final model. It should be noted that training the Wide model and the Deep model at the same time does not mean model fusion, but the weighted sum of the results of the two models servers as the final prediction result:

$$P(Y = 1 \mid x) = \sigma(w_{wide}^T [x, \emptyset(x)] + w_{deep}^T a^{(lf)} + b)$$

In the above Sigmoid function, $W_{wide}$ is the weight of the Wide part, x is the original feature vector, Φ (x) is the cross feature, for example, the new feature combined after one-hot encoding, $W_{deep}$ is the weight output by the last activation layer of the neural network of the Deep part, I represents the hidden layer, $f$ represents the activation function, a is the input feature, and b is the bias term.

Model training uses joint training. Compared with the individual training of a single model in integrated learning, the model is only fused in the final prediction stage, while model fusion is performed in the training stage of joint training. The training error is fed back to both the Wide model and the Deep model to update the weight. Therefore, the Wide model focuses on the cross product of discrete features, and non-linear transformation is performed on the original feature to generate the memory of feature interaction. The Deep model focuses on generalization, and the deep neural network uses low-dimensional dense features and requires only a small amount of feature engineering to better generalize the feature combination not appeared in the training sample, thereby improving the generalization ability of the model. After the model is trained, the model is deployed to the question recommendation scenario.

It should be noted that more information about the Wide&Deep model may be referred to other related references, and the above description of the present disclosure is only a schematic introduction.

For example, in an example of the present disclosure, in the question recommendation method 10, the ranking model (i.e., the Wide&Deep model) is adopted to compose the input feature vector by basic user information, the plurality of candidate questions, and at least one similarity feature. Each of the candidate questions included in the candidate question set returns a score. According to the value of the score, the candidate questions are sorted to obtain the question sequence. For example, in an example, the scores are sorted from high to low. For example, in another example, the scores are sorted from low to high. It should be noted that the scores can also be sorted in other orders, and the embodiments of the present disclosure are not specifically limited in this aspect.

In step S170, based on an order of the question sequence, at least one candidate question in the question sequence is recommended to the user. For example, in an example, the question sequence is sorted according to the order of the scores from high to low, and the first N candidate questions in the question sequence are recommended to the user, where N is an integer greater than or equal to 1. For example, the first 5 (or other numerical values) candidate questions in the question sequence are selected as the final recommendation questions and recommended to the user.

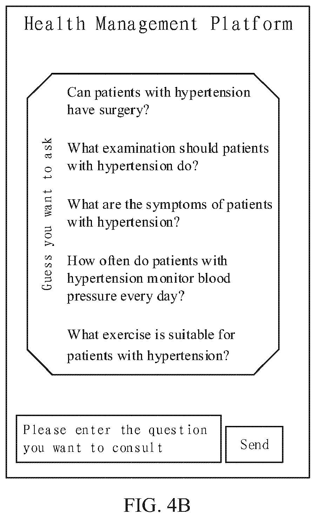

FIG. 4B illustrates a user interface diagram of a recommendation question provided to a user by a certain platform (for example, a health management platform). For example, in an example, as illustrated in FIG. 4B, before the user enters the question that the user wants to consult in the text input box, the health management platform has already recommended 5 questions (for example, "Can patients with hypertension have surgery?", "What examination should patients with hypertension do?", "What are the symptoms of patients with hypertension?", "How often do patients with hypertension monitor blood pressure every day?", and "What exercise is suitable for patients with hypertension?") to the user based on the above question recommendation method. It should be noted that there are various ways to provide the final recommendation questions to the user, and the embodiments of the present disclosure are not specifically limited in this aspect.

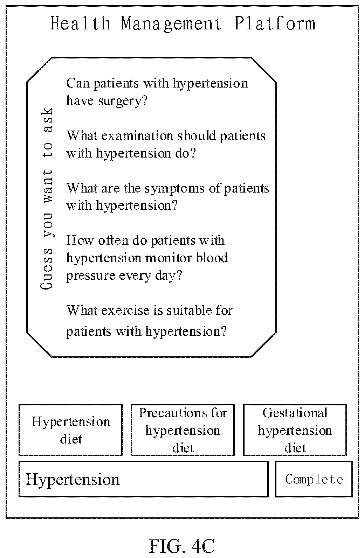

For example, in an embodiment, when the user is inputting the question that the user wants to consult into the input text box, the platform can match the related question in the data knowledge base according to the text information input by the user through, for example, entity recognition, keyword matching, or other suitable methods. These questions are displayed at the top of the input text box in the user interface. For example, as illustrated in FIG. 4C, when the user enters the word "hypertension" in the input text box, recommendation questions of "hypertension diet," "precautions for hypertension diet" and "gestational hypertension diet" appear at the top of the input text box, and the embodiments of the present disclosure are not specifically limited in this aspect, which can be adjusted according to actual conditions.

It should be noted that some steps of step S110 to step S170 may be selectively executed according to actual conditions, which are not specifically limited in the embodiments of the present disclosure.

For example, in an embodiment, when the same user uses a certain platform multiple times, if the basic information has not changed, step S110 to step S130 can be omitted, and the pre-stored candidate question set of the user can be directly retrieved. For example, in an example, when a user uses a certain platform (for example, a health management platform) for the first time, the platform has generated a corresponding candidate question set for the user, which is saved in the database associated with the platform. When the user uses the platform for the second time, step S110 to step S130 may not be repeated to obtain the candidate question set of the user, but the candidate question set of the user can be obtained by retrieving the candidate question set previously stored in the associated database. The embodiments of the present disclosure are not specifically limited in this aspect, which may be adjusted according to actual conditions.

The question recommendation method 10 provided by at least one embodiment of the present disclosure not only effectively avoids the question of inappropriate feedback answers due to unclear expression of the patient, but also provides personalized question recommendation regarding to individual factors of the user (for example, basic user information, user behavior data, etc.), so that the user can more pertinently learn the knowledge related to health conditions. Moreover, the ranking model (i.e., the Wide&Deep model) provides fusion of various features, such as basic user information, the output of each similarity matching model, and the like, so as to make the question recommendation relevant, personalized, and diversified, and simultaneously pay attention to the final feedback sequence, thereby achieving a more user-friendly effect.

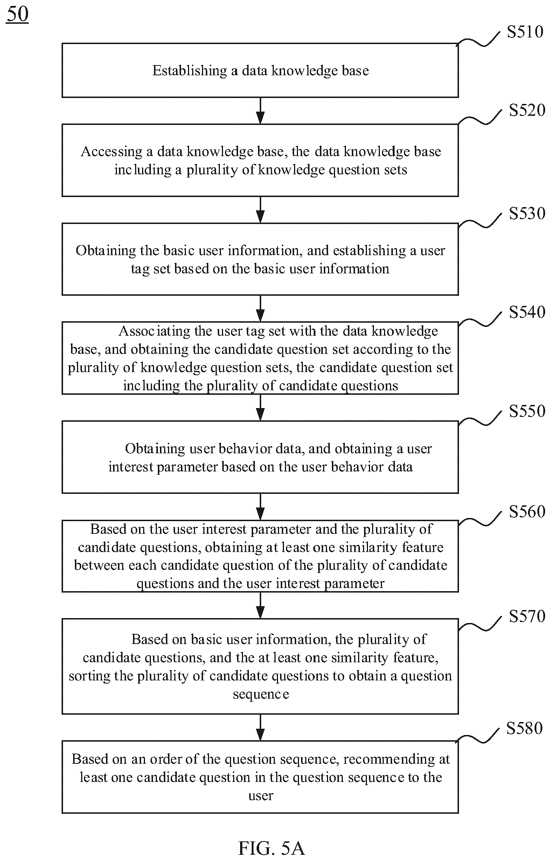

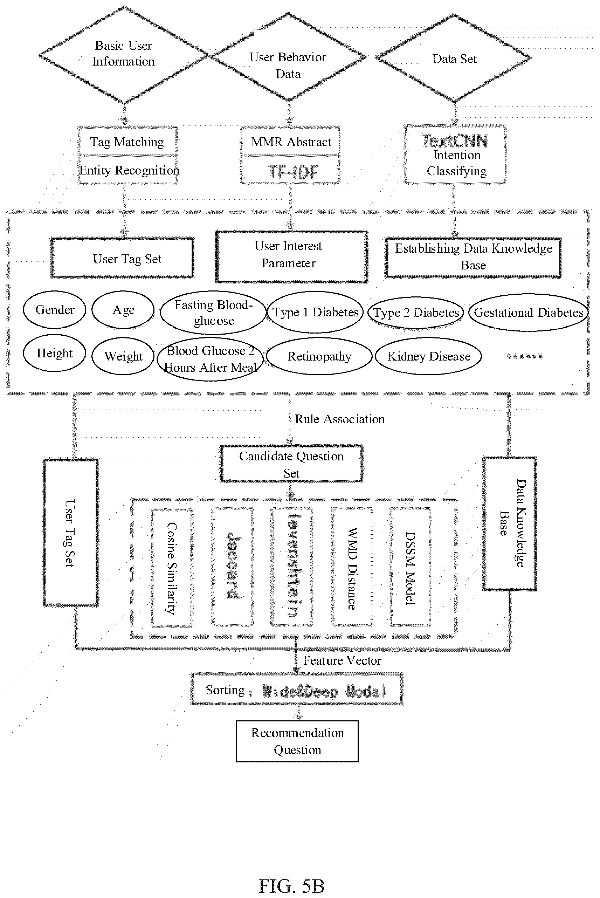

FIG. 5A is an exemplary flowchart of another question recommendation method provided by at least one embodiment of the present disclosure, and FIG. 5B is a schematic block diagram of the question recommendation method as illustrated in FIG. 5A provided by at least one embodiment of the present disclosure. As illustrated in FIG. 5A, the question recommendation method 50 includes step S510 to step S580. For example, step S510 to step S580 may be executed in sequence, or may be executed in other adjusted order. For example, step S520 may be executed first and then step S530 may be executed, or step S530 may be executed first and then step S520 may be executed. For another example, part or all of the operations in step S510 to step S580 can also be executed in parallel. For example, step S520 and step S530 can be executed in parallel. The embodiments of the present disclosure do not limit the execution order of each step, which can be adjusted according to actual conditions. For example, step S510 to step S580 can be implemented by a server or a local terminal, which is not limited in the embodiments of the present disclosure. For example, in some examples, the implementation of the question recommendation method 50 may selectively execute some of the steps in step S510 to step S580, or may execute some additional steps in addition to step S510 to step S580, and the embodiments of the present disclosure are not specifically limited in this aspect.

Referring to FIG. 5A and FIG. 1B, step S520 to step S580 included in the question recommendation method 50 illustrated in FIG. 5A are basically the same as step S110 to step S170 included in the question recommendation method 10 illustrated in FIG. 1B. Therefore, descriptions of step S520 to step S580 may be referred to the relevant descriptions of step S110 to step S170 in FIG. 1B, and details are not described herein again.

Compared with the question recommendation method 10 illustrated in FIG. 1B, FIG. 5A further includes step S510, that is, establishing a data knowledge base. For example, in an embodiment of the present disclosure, the data set can be captured from the network, and the data set can be classified according to intention, so as to form multiple knowledge question sets, thereby establishing the data knowledge base.

For example, in an example, a web crawler can be used to grab the data collection containing a large amount of data from the network such as the Internet. For example, the web crawler, also known as the web spider, is a program or script that automatically crawls information on the World Wide Web according to certain rules.

For example, in an embodiment of the present disclosure, when the above-mentioned question recommendation method 50 is applied to a medical intelligent question answering scenario, the question recommendation method 50 can be used to recommend the questions related to a certain disease, and the data set can be based on at least one of a group consisting of the diagnosis data set between the doctor and the patient, the hot issue related to the disease, and the reward question. For example, in an example, the reward question may be a question that requires payment for consultation on certain websites (for example, www.xywy.com, www.39.net, etc.), and the embodiments of the present disclosure are not specifically limited in this aspect.

For example, the TF-IDF method may be used to extract the high-frequency keyword from the data set, such as the "symptoms," "treatment," "blood glucose," "diet," "medication," "examination," "insulin," "diabetic foot," etc. According to the high-frequency keyword, a large amount of data from the data set can be classified based on intention, which can facilitate the construction of the data knowledge base. For example, the deep learning algorithm Text-CNN is used to classify the data set based on intention, and standard questions and corresponding extended questions and standard answers are sorted (for example, manually sorted) under each intention, thereby establishing the complete data knowledge base containing basic health knowledge related to chronic diseases, such as diabetes, hypertension, and the like. For example, in an example, based on the extracted high-frequency keyword, multiple types of intentions can be manually determined. For example, the type of intention can be manually determined according to the degree of attention of people, the frequency of occurrence of keywords, etc., and the embodiments of the present disclosure are not specifically limited in this aspect. For example, in an example, the following intention categories are manually determined: diet, exercise, medication, examination, complications, surgery, treatment, symptoms, etc., and the embodiments of the present disclosure are not specifically limited in this aspect. Then, corresponding data (for example, the questions matching this type of intention) can be manually organized under each category of intention and used as training data for model training by using the deep learning algorithm Text-CNN. Then a large amount of data from the data set may be input into the trained model, and the model outputs the intention category corresponding to each data, so as to realize the intention classification of a large amount of data. In order to improve the accuracy of intention classification, standard questions and corresponding extended questions and standard answers can be manually filtered, sorted and supplemented under each intention. For example, when the question recommendation method 50 is executed for the first time, step S510 is executed to establish the data knowledge base, and the data knowledge base is stored in a server, a memory, or a database. When the question recommendation method 50 is executed again later, step S510 can be omitted, and the data knowledge base can be directly accessed, thereby improving the processing efficiency. For example, step S510 can be executed once in a while, or the data knowledge base can be updated in other suitable ways, so that the update and optimization of the data knowledge base can be realized, and the candidate questions obtained in the subsequent steps can be more relevant to user needs and more relevant to the cognition level of current society.

The technical effects achieved by the question recommendation method 50 illustrated in FIG. 5A are similar to the technical effects achieved by the question recommendation method 10 described above in conjunction with FIG. 1B, and details are not described herein again. The descriptions of each block diagram illustrated in FIG. 5B may be referred to the above detailed descriptions of each step in FIG. 5A and FIG. 1B, and details are not described herein again.

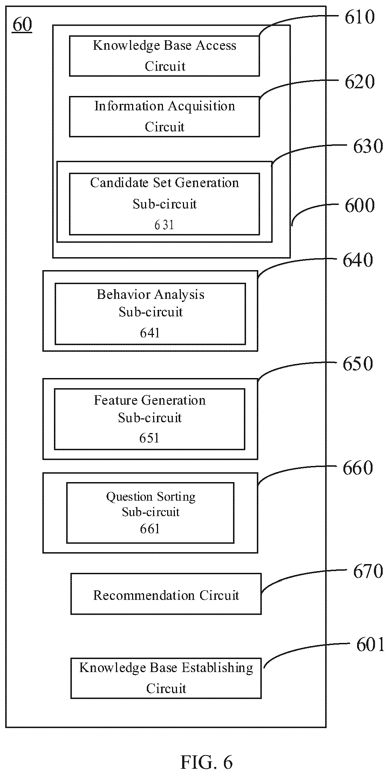

At least one embodiment of the present disclosure further provides a question recommendation device. FIG. 6 is a schematic block diagram of a question recommendation device provided by at least one embodiment of the present disclosure. As illustrated in FIG. 6, the question recommendation device 60 includes: a set acquisition module, a behavior analysis module, a feature generation module, a question sorting module, and a recommendation module. These modules can be implemented by software, hardware, firmware, or any combination thereof, and for example, can be implemented as a set acquisition circuit 600, a behavior analysis circuit 640, a feature generation circuit 650, a question sorting circuit 660, and a recommendation circuit 670, respectively.

For example, in an example, the set acquisition circuit 600 is configured to obtain a candidate question set of a user, and the candidate question set includes a plurality of candidate questions. For example, the set acquisition circuit 600 includes a knowledge base access circuit 610, an information acquisition circuit 620, and a candidate set generation circuit 630. For example, the knowledge base access circuit 610 is configured to access a data knowledge base including a plurality of knowledge question sets. For example, the information acquisition circuit 620 is configured to obtain the basic user information and establish a user tag set based on the basic user information. For example, the candidate set generation circuit 630 is configured to associate the user tag set with the data knowledge base and obtain the candidate question set according to the plurality of knowledge question sets, and the candidate question set includes a plurality of candidate questions. For example, the behavior analysis circuit 640 is configured to obtain user behavior data and obtain a user interest parameter based on the user behavior data. For example, the feature generation circuit 650 is configured to obtain at least one similarity feature between each candidate question of the plurality of candidate questions and the user interest parameter based on the user interest parameter and the plurality of candidate questions. For example, the question sorting circuit 660 is configured to sort the plurality of candidate questions to obtain a question sequence based on basic user information, the plurality of candidate questions, and the at least one similarity feature. For example, the recommendation circuit 670 is configured to recommend at least one candidate question in the question sequence to the user based on an order of the question sequence.

For example, the specific operations, which the knowledge base access circuit 610, the information acquisition circuit 620, the candidate set generation circuit 630, the behavior analysis circuit 640, the feature generation circuit 650, the question sorting circuit 660, and the recommendation circuit 670 are configured to perform, may be with reference to the related descriptions of the question recommendation methods 10 and 50 provided by at least one embodiment of the present disclosure described above, and details are not described herein again.

For example, in at least one embodiment of the present disclosure, the question sorting circuit 660 in the question recommendation device 60 includes a question sorting sub-circuit 661. The question sorting sub-circuit 661 is configured to, by using a ranking model, combine the basic user information, the plurality of candidate questions, and the at least one similarity feature to form an input feature vector of the ranking model, obtain a score corresponding to each candidate question of the plurality of candidate questions, and sort the plurality of candidate questions according to a value of the score corresponding to each candidate question of the plurality of candidate questions, so as to obtain the question sequence.

For example, the specific operations, which the question sorting sub-circuit 661 is configured to perform, may be with reference to the related descriptions of the question recommendation methods 10 and 50 provided by at least one embodiment of the present disclosure described above, and details are not described herein again.

For example, in at least one embodiment of the present disclosure, the question recommendation device 60 further includes a knowledge base establishing circuit 601. The knowledge base establishing circuit 601 is configured to retrieve a data set from network and classify the data set according to intention to form the plurality of knowledge question sets, so as to establish the data knowledge base.

For example, the specific operations, which the knowledge base establishment circuit 601 is configured to perform, may be with reference to the related descriptions of the question recommendation method 50 provided by at least one embodiment of the present disclosure described above, and details are not described herein again.

For example, in at least one embodiment of the present disclosure, the candidate set generation circuit 630 in the question recommendation device 60 includes a candidate set generation sub-circuit 631. The candidate set generation sub-circuit 631 is configured to establish a mapping relationship between the user tag set and the standard question in the data knowledge base, match the user tag set with the standard question in the data knowledge base, and combine the knowledge question set corresponding to the matched standard question to form the candidate question set.

For example, the specific operations, which the candidate set generation sub-circuit 631 is configured to perform, may be with reference to the related descriptions of the question recommendation methods 10 and 50 provided by at least one embodiment of the present disclosure described above, and details are not described herein again.

For example, in at least one embodiment of the present disclosure, the behavior analysis circuit 640 in the question recommendation device 60 includes a behavior analysis sub-circuit 641. The behavior analysis sub-circuit 641 is configured to analyze the user behavior data and convert a question clicked by the user or a word or sentence which the user is interested in into the user interest parameter.

For example, the specific operations, which the behavior analysis sub-circuit 641 is configured to perform, may be with reference to the related descriptions of the question recommendation methods 10 and 50 provided by at least one embodiment of the present disclosure described above, and details are not described herein again.

For example, in at least one embodiment of the present disclosure, the feature generation circuit 650 in the question recommendation device 60 includes a feature generation sub-circuit 651. The feature generation sub-circuit 651 is configured to, by using at least one similarity matching model, obtain the at least one similarity feature between each candidate question and the user interest parameter based on the user interest parameter and the plurality of candidate questions.

For example, the specific operations, which the feature generation sub-circuit 651 is configured to perform, may be with reference to the related descriptions of the question recommendation methods 10 and 50 provided by at least one embodiment of the present disclosure described above, and details are not described herein again.

It should be noted that the set acquisition circuit 600, the knowledge base access circuit 610, the information acquisition circuit 620, the candidate set generation circuit 630, the behavior analysis circuit 640, the feature generation circuit 650, the question sorting circuit 660, the recommendation circuit 670, the feature generation sub-circuit 651, the behavior analysis sub-circuit 641, the candidate set generation sub-circuit 631, the question sorting sub-circuit 661, and the knowledge base establishing circuit 601 in the embodiments of the present disclosure can be implemented as hardware such as a processor, a controller, or the like, software that can implement related functions, or a combination of hardware and software. The embodiments of the present disclosure do not limit the specific implementation manners.

It should also be noted that, in the embodiments of the present disclosure, the question recommendation device 60 may further include more circuits, and is not limited to the set acquisition circuit 600, the knowledge base access circuit 610, the information acquisition circuit 620, the candidate set generation circuit 630, the behavior analysis circuit 640, the feature generation circuit 650, the question sorting circuit 660, the recommendation circuit 670, the feature generation sub-circuit 651, the behavior analysis sub-circuit 641, the candidate set generation sub-circuit 631, the question sorting sub-circuit 661, and the knowledge base establishing circuit 601 described above, which can be determined according to actual needs, and the embodiments of the present disclosure are not limited in this aspect.

It should be understood that the question recommendation device 60 provided by the embodiments of the present disclosure can implement the aforementioned question recommendation methods 10 and 50, and can also achieve similar technical effects to those of the aforementioned question recommendation methods 10 and 50, and details are not described herein again.

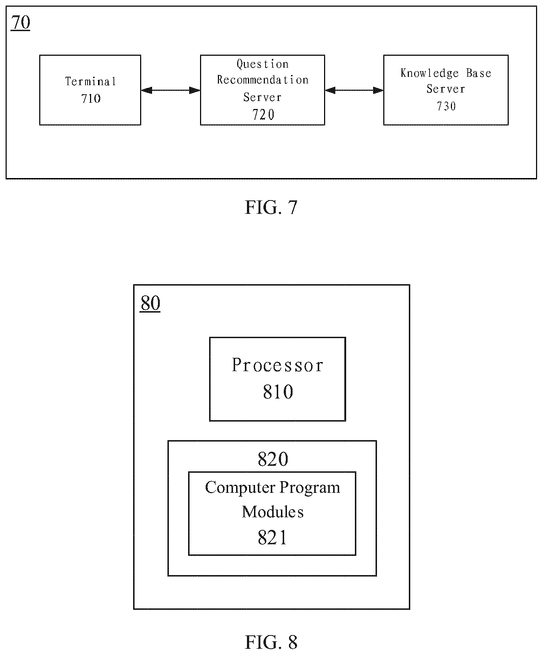

At least one embodiment of the present disclosure further provides a question recommendation system. FIG. 7 is a schematic block diagram of a question recommendation system provided by at least one embodiment of the present disclosure. As illustrated in FIG. 7, the question recommendation system 70 includes a terminal 710 and a question recommendation server 720, and the terminal 710 and the question recommendation server 720 are in signal connection. The terminal 710 is configured to send request data to the question recommendation server 720. The question recommendation server 720 is configured to, in response to the request data, access the data knowledge base which includes a plurality of knowledge question set, obtain a candidate question set of a user which comprises a plurality of candidate questions, obtain user behavior data, and obtain a user interest parameter based on the user behavior data, based on the user interest parameter and the plurality of candidate questions, obtain at least one similarity feature between each candidate question of the plurality of candidate questions and the user interest parameter, and based on basic user information, the plurality of candidate questions, and the at least one similarity feature, sort the plurality of candidate questions to obtain a question sequence. For example, the terminal 710 is further configured to display first N candidate question(s) in the question sequence, and N is an integer greater than or equal to 1.

For example, the above-mentioned operations, which the question recommendation server 720 is configured to perform, may be with reference to the question recommendation methods 10 and 50 provided by at least one embodiment of the present disclosure, and details are not described herein again.

For example, in an example, the terminal 710 included in the question recommendation system 70 may be implemented as a client terminal (such as a mobile phone, a computer, etc.), and the question recommendation server 720 may be implemented as a server terminal (such as a server).

For example, in an example, as illustrated in FIG. 7, in addition to the terminal 710 and the question recommendation server 720, the question recommendation system 70 may further include a knowledge base server 730 storing a data knowledge base. The knowledge base server 730 is in signal connection with the question recommendation server 720, and is configured to, in response to the request information of the question recommendation server 720, return data corresponding to the request information in the data knowledge base to the question recommendation server 720. It should be noted that in the case where the question recommendation system 70 does not include the knowledge base server 730, the data in the data knowledge base can be directly stored in the question recommendation server 720 or stored in another storage device provided separately, or the data knowledge base may be independently established by the question recommendation server 720 and stored in the question recommendation server 720 or in another storage device provided separately, which is not specifically limited in the embodiments of the present disclosure.

The question recommendation system 70 provided by at least one embodiment of the present disclosure can implement the question recommendation methods 10 and 50 provided by the foregoing embodiments, and can also achieve similar technical effects as those of the question recommendation methods 10 and 50 provided by the foregoing embodiments, and details are not described herein again.

At least one embodiment of the present disclosure further provides an electronic device. FIG. 8 is a schematic diagram of an electronic device provided by at least one embodiment of the present disclosure. For example, as illustrated in FIG. 8, the electronic device 80 includes a processor 810 and a memory 820. The memory 820 includes one or more computer program modules 821. The one or more computer program modules 821 are stored in the memory 820 and configured to be executed by the processor 810, and the one or more computer program modules 821 include instructions for performing the question recommendation method provided by at least one embodiment of the present disclosure. When the instructions are executed by the processor 810, one or more steps in the question recommendation method provided by at least one embodiment of the present disclosure can be performed. The memory 820 and the processor 810 may be interconnected by a bus system and/or other forms of connection mechanisms (not illustrated).

For example, the memory 820 and the processor 810 may be provided on the server terminal (or the cloud), for example, set in the aforementioned question recommendation server 720, so as to execute one or more steps in the question recommendation method described in FIG. 1A, FIG. 1B, and FIG. 5A.

For example, the processor 810 may be a central processing unit (CPU), a digital signal processor (DSP), or other forms of processing units with data processing capabilities and/or program executing capabilities, such as a field programmable gate array (FPGA), etc. For example, the central processing unit (CPU) may be an X86 or ARM architecture, etc. The processor 810 may be a general-purpose processor or a special-purpose processor, and may control other components in the electronic device 80 to perform desired functions.

For example, the memory 820 may include any combination of one or more computer program products, and the computer program product may include various forms of computer-readable storage media, such as a volatile memory and/or a non-volatile memory. For example, the volatile memory may include a random access memory (RAM) and/or a cache memory. For example, the non-volatile memory may include a read-only memory (ROM), a hard disk, an erasable programmable read-only memory (EPROM), a portable compact-disk read-only memory (CD-ROM), a USB memory, a flash memory, etc. One or more computer program modules 821 may be stored in the computer-readable storage medium, and the processor 810 may run the one or more computer program modules 821 to implement various functions of the electronic device 80. The computer-readable storage medium may also store various application programs and various data, such as various data used and/or generated by the application programs. The specific functions and technical effects of the electronic device 80 may be with reference to the above descriptions of the question recommendation method, and details are not described herein again.

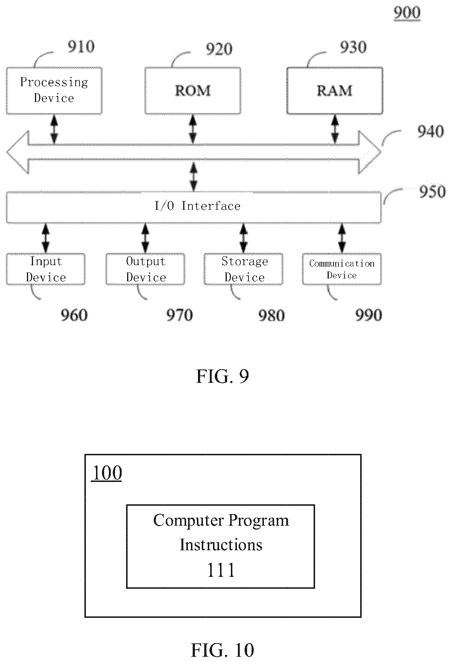

FIG. 9 is a schematic block diagram of a terminal provided by at least one embodiment of the present disclosure. For example, in at least one embodiment of the present disclosure, the terminal is the display terminal 900, which may be applied to the question recommendation method provided by the embodiments of the present disclosure. For example, the display terminal 900 may provide user access logs reflecting user behavior data (for example, access logs recorded by applications such as a browser running in the system through cookies, etc.), and may display at least one candidate question recommended to the user. It should be noted that the terminal illustrated in FIG. 9 as the display terminal 900 is only an example, which does not bring any limitation to the functions and scope of use of the embodiments of the present disclosure.

As illustrated in FIG. 9, the display terminal 900 may include a processing device (such as a central processing unit, a graphics processor, etc.) 910, which may execute various appropriate actions and processing according to the program stored in the read-only memory (ROM) 920 or the program loaded from the storage device 980 into the random access memory (RAM) 930. Various programs and data required for the operations of the display terminal 900 are also stored in the RAM 930. The processing device 910, the ROM 920, and the RAM 930 are connected to each other through a bus 940. An input/output (I/O) interface 950 is also connected to the bus 940.

Generally, the following devices can be connected to the I/O interface 950: the input device 960 including such as a touch screen, a touch pad, a keyboard, a mouse, a camera, a microphone, an accelerometer, a gyroscope, or the like; the output device 970 including such as a liquid crystal display (LCD), a speaker, a vibration device, or the like; the storage device 980 including such as a magnetic tape, a hard disk, or the like; and the communication device 990. The communication device 990 may allow the display terminal 900 to perform wireless or wired communication with other electronic devices to exchange data. Although FIG. 9 illustrates the display terminal 900 having various devices, it should be understood that the display terminal 900 is not required to implement or have all the illustrated devices, and the display terminal 900 may be alternatively implemented or have more or fewer devices.

At least one embodiment of the present disclosure further provides a non-volatile readable storage medium. FIG. 10 is a schematic block diagram of a non-volatile readable storage medium 100 provided by at least one embodiment of the present disclosure. For example, as illustrated in FIG. 10, the non-volatile readable storage medium 100 includes computer program instructions 111 stored thereon. When the computer program instructions 111 are executed by the processor, one or more steps in the question recommendation method 10 or 50 provided by at least one embodiment of the present disclosure are executed.

For example, the storage medium may be any combination of one or more computer-readable storage media. For example, one computer-readable storage medium contains computer-readable program codes for obtaining the candidate question set of the user, another computer-readable storage medium contains computer-readable program codes for obtaining user behavior data and obtaining the user interest parameter based on the user behavior data, still another computer-readable storage medium contains computer-readable program codes for obtaining at least one similarity feature between each candidate question of the plurality of candidate questions and the user interest parameter based on the user interest parameter and the plurality of candidate questions, and further still another computer-readable storage medium contains computer-readable program codes for sorting the plurality of candidate questions to obtain a question sequence based on basic user information, the plurality of candidate questions, and the at least one similarity feature. Certainly, each of the above-mentioned program codes can also be stored in the same computer-readable medium, which is not limited in the embodiments of the present disclosure. For example, when the program codes are read by a computer, the computer can execute the program codes stored in the computer storage medium and execute such as the question recommendation method provided by any one of the embodiments of the present disclosure.

For example, the storage medium may include a memory card of a smart phone, a storage component of a tablet computer, a hard disk of a personal computer, a random access memory (RAM), a read-only memory (ROM), an erasable programmable read-only memory (EPROM), a portable compact-disk read-only memory (CD-ROM), a flash memory, or any combination of the foregoing storage media, and may also be other suitable storage media. For example, the readable storage medium may also be the memory 820 in FIG. 8, and the relevant descriptions may be referred to the foregoing content, which are not described herein again.

It should be noted that the storage medium 100 can be applied to the question recommendation server 720, and the technician can make a selection according to specific scenarios, which is not limited herein.

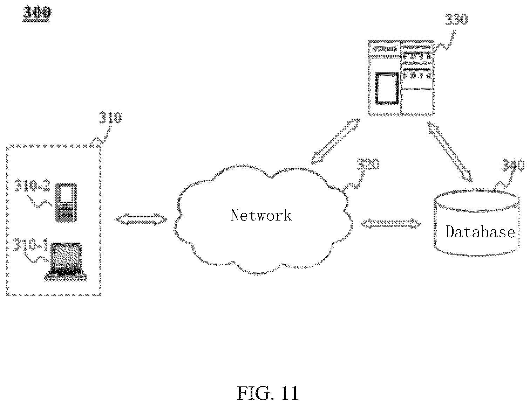

FIG. 11 illustrates an exemplary scene diagram of a question recommendation system provided by at least one embodiment of the present disclosure. As illustrated in FIG. 11, the question recommendation system 300 may include a user terminal 310, a network 320, a server 330, and a database 340.

For example, the user terminal 310 may be a computer 310-1 or a portable terminal 310-2 illustrated in FIG. 11. It can be understood that the user terminal may also be any other type of electronic device capable of receiving, processing and displaying data, which may include but is not limited to a desktop computer, a notebook computer, a tablet computer, a smart home device, a wearable device, an automotive electronic device, a medical electronic device, etc.

For example, the network 320 may be a single network, or a combination of at least two different networks. For example, the network 320 may include, but is not limited to, one or a combination of a local area network, a wide area network, a public network, a private network, the Internet, a mobile communication network, and the like.

For example, the server 330 may be a single server or a server group, and each server in the server group is connected through a wired network or a wireless network. For example, the wired network may use the twisted pair, coaxial cable, optical fiber transmission, or the like for communication, and the wireless network may use the 3G/4G/5G mobile communication network, Bluetooth, Zigbee, WiFi, or the like for communication. The present disclosure does not limit the types and functions of the network here. The server group can be centralized, such as a data center, or can also be distributed. The server may be local or remote. For example, the server 330 may be a general-purpose server or a dedicated server, and may be a virtual server or a cloud server.

For example, the database 340 may be used to store various data used, generated, and output from the work of the user terminal 310 and the server 330. The database 340 may be connected or communicated with the server 330 or a part of the server 330 via the network 320 or directly connected or communicated with the server 330, or may be connected or communicated with the server 330 through a combination of the above two methods. In some embodiments, the database 340 may be a stand-alone device. In other embodiments, the database 340 may also be integrated in at least one of the user terminal 310 and the server 340. For example, the database 340 may be set on the user terminal 310 or on the server 340. For another example, the database 340 may also be distributed, a part of the database 340 is set on the user terminal 310, and the other part of the database 340 is set on the server 340.

For example, in an example, firstly, the user terminal 310 (for example, the user's mobile phone) may send the request data to the server 330 via the network 320 or other technologies (for example, Bluetooth communication, infrared communication, etc.). Next, the server 330 obtains the candidate question set of the user in response to the request data, and the candidate question set includes candidate questions. Then, the server 330 obtains user behavior data, and obtains the user interest parameter based on the user behavior data. For example, the user behavior data is transmitted from the user terminal 310 to the server 330 via the network 320. Then, the server 330 obtains at least one similarity feature between each candidate question of the candidate questions and the user interest parameter based on the user interest parameter and the candidate questions. Then, the server 330 sorts the candidate questions based on the basic user information, the candidate questions, and the at least one similarity feature, so as to obtain the question sequence, and then sends the first N candidate questions in the question sequence to the user terminal 310 via the network 320 or other technologies (for example, Bluetooth communication, infrared communication, etc.). Finally, the user terminal 310 receives and displays the first N candidate questions from the server 330.

In the present disclosure, the term "plurality" refers to two or more than two unless specifically defined otherwise.

Those skilled in the art may easily think of other embodiments of the present disclosure after considering the specification and practicing the disclosure disclosed herein. The present disclosure is intended to cover any variations, uses, or adaptive changes of the present disclosure. These variations, uses, or adaptive changes follow the general principles of the present disclosure and include common knowledge or conventional technical means in the technical field that are not disclosed in the present disclosure. The description and the embodiments are exemplary only, and the scope and spirit of the present disclosure are pointed out by the following claims.

It should be understood that the present disclosure is not limited to the precise structure that has been described above and illustrated in the drawings, and various modifications and changes can be made without departing from the scope. The protection scope of the present disclosure should be based on the protection scope of the claims.

**Claims**

1. A question recommendation method, comprising:

   obtaining a candidate question set of a user, wherein the candidate question set comprises a plurality of candidate questions;
   obtaining user behavior data, and obtaining a user interest parameter based on the user behavior data;
   based on the user interest parameter and the plurality of candidate questions, obtaining at least one similarity feature between each candidate question of the plurality of candidate questions and the user interest parameter;
   based on basic user information, the plurality of candidate questions, and the at least one similarity feature, sorting the plurality of candidate questions to obtain a question sequence; and
   based on an order of the question sequence, recommending at least one candidate question in the question sequence to the user.

2. The question recommendation method according to claim 1, wherein based on the basic user information, the plurality of candidate questions, and the at least one similarity feature, sorting the plurality of candidate questions to obtain the question sequence, comprises:
   by using a ranking model, combining the basic user information, the plurality of candidate questions, and the at least one similarity feature to form an input feature vector of the ranking model, obtaining a score corresponding to each candidate question of the plurality of candidate questions, and sorting the plurality of candidate questions according to a value of the score corresponding to each candidate question of the plurality of candidate questions, so as to obtain the question sequence.

3. The question recommendation method according to claim 1 or 2, wherein based on the user interest parameter and the plurality of candidate questions, obtaining at least one similarity feature between each candidate question of the plurality of candidate questions and the user interest parameter, comprises:
   by using at least one similarity matching model, obtaining the at least one similarity feature between each candidate question and the user interest parameter based on the user interest parameter and the plurality of candidate questions.

4. The question recommendation method according to claim 3, wherein the at least one similarity matching model comprises at least one of a group consisting of a cosine similarity model, a Jaccard similarity model, an edit distance similarity model, a word mover's distance similarity model, and a deep structured semantic similarity model.

5. The question recommendation method according to claim 2, wherein the ranking model comprises a Wide&Deep model.

6. The question recommendation method according to any one of claims 1 to 5, wherein obtaining the candidate question set of the user comprises:

accessing a data knowledge base, wherein the data knowledge base comprises a plurality of knowledge question sets;

obtaining the basic user information, and establishing a user tag set based on the basic user information; and associating the user tag set with the data knowledge base, and obtaining the candidate question set according to the plurality of knowledge question sets.

7. The question recommendation method according to claim 6, wherein the user tag set comprises a multi-level tag set, the multi-level tag set comprises tags of a plurality of levels, and tags of different levels are of different types.

8. The question recommendation method according to claim 7, wherein the question recommendation method is used to recommend a question related to disease,

a first-level tag of the multi-level tag set is an age group,
a second-level tag of the multi-level tag set is a time period,
a third-level tag of the multi-level tag set is a type of disease, and
a fourth-level tag of the multi-level tag set is a complication.

9. The question recommendation method according to any one of claims 6 to 8, wherein each of the plurality of knowledge question sets comprises:
a standard question, a standard answer corresponding to the standard question, and an extended question corresponding to the standard question.

10. The question recommendation method according to any one of claims 6 to 9, wherein obtaining the candidate question set of the user further comprises:
establishing the data knowledge base.

11. The question recommendation method according to claim 10, wherein establishing the data knowledge base comprises:
retrieving a data set from a network, and classifying the data set according to intention to form the plurality of knowledge question sets, so as to establish the data knowledge base.

12. The question recommendation method according to claim 11, wherein the question recommendation method is used to recommend a question related to disease, and the data set is based on at least one of a group consisting of a medical consultation data set between a doctor and a patient, a hotspot question associated with the disease, and a reward question associated with the disease.

13. The question recommendation method according to claim 9, wherein associating the user tag set with the data knowledge base, and obtaining the candidate question set according to the plurality of knowledge question sets, comprises:

establishing a mapping relationship between the user tag set and the standard question in the data knowledge base,
matching the user tag set with the standard question in the data knowledge base, and
combining the knowledge question set corresponding to a matched standard question to form the candidate question set.

14. The question recommendation method according to any one of claims 1 to 5, wherein obtaining the candidate question set of the user comprises:
retrieving the candidate question set of the user stored beforehand.

15. The question recommendation method according to any one of claims 1 to 14, wherein obtaining the user interest parameter based on the user behavior data comprises:
analyzing the user behavior data, and converting a question clicked by the user or a word or sentence which the user is interested in into the user interest parameter.

16. A question recommendation device, comprising:

a set acquisition circuit, configured to obtain a candidate question set of a user, wherein the candidate question set comprises a plurality of candidate questions;
a behavior analysis circuit, configured to obtain user behavior data and obtain a user interest parameter based on the user behavior data;
a feature generation circuit, configured to obtain at least one similarity feature between each candidate question of the plurality of candidate questions and the user interest parameter based on the user interest parameter and the plurality of candidate questions;
a question sorting circuit, configured to sort the plurality of candidate questions to obtain a question sequence based on basic user information, the plurality of candidate questions, and the at least one similarity feature; and
a recommendation circuit, configured to recommend at least one candidate question in the question sequence to the user based on an order of the question sequence.

17. The question recommendation device according to claim 16, wherein the question sorting circuit comprises:
a question sorting sub-circuit, configured to, by using a ranking model, combine the basic user information, the plurality of candidate questions, and the at least one similarity feature to form an input feature vector of the ranking model, obtain a score corresponding to each candidate question of the plurality of candidate questions, and sort the plurality of candidate questions according to a value of the score corresponding to each candidate question of the plurality of candidate questions, so as to obtain the question sequence.

18. The question recommendation device according to claim 16 or 17, wherein the set acquisition circuit comprises:

a knowledge base access circuit, configured to access a data knowledge base, wherein the data knowledge base comprises a plurality of knowledge question sets;
an information acquisition circuit, configured to obtain the basic user information and establish a user tag set based on the basic user information; and
a candidate set generation circuit, configured to associate the user tag set with the data knowledge base and obtain the candidate question set according to the plurality of knowledge question sets, wherein the candidate question set comprises the plurality of candidate questions.

19. The question recommendation device according to claim 18, wherein the set acquisition circuit further comprises:
a knowledge base establishing circuit, configured to retrieve a data set from a network and classify the data set according to intention to form the plurality of knowledge question sets, so as to establish the data knowledge base.

20. The question recommendation device according to claim 18 or 19, wherein the candidate set generation circuit comprises:
a candidate set generation sub-circuit, configured to establish a mapping relationship between the user tag set and the standard question in the data knowledge base, match the user tag set with the standard question in the data knowledge base, and combine the knowledge question set corresponding to a matched standard question to form the candidate question set.

21. The question recommendation device according to any one of claims 16 to 20, wherein the behavior analysis circuit comprises:
a behavior analysis sub-circuit, configured to analyze the user behavior data and convert a question clicked by the user or a word or sentence which the user is interested in into the user interest parameter.

22. The question recommendation device according to any one of claims 16 to 21, wherein the feature generation circuit comprises:
a feature generation sub-circuit, configured to, by using at least one similarity matching model, obtain the at least one similarity feature between each candidate question and the user interest parameter based on the user interest parameter and the plurality of candidate questions.

23. A question recommendation system, comprising a terminal and a question recommendation server,

wherein the terminal is configured to send request data to the question recommendation server;
the question recommendation server is configured to:
in response to the request data:

obtain a candidate question set of a user, wherein the candidate question set comprises a plurality of candidate questions;

obtain user behavior data, and obtain a user interest parameter based on the user behavior data;

based on the user interest parameter and the plurality of candidate questions, obtain at least one similarity feature between each candidate question of the plurality of candidate questions and the user interest parameter; and

based on basic user information, the plurality of candidate questions, and the at least one similarity feature, sort the plurality of candidate questions to obtain a question sequence; and

the terminal is further configured to display first N candidate questions in the question sequence, and N is an integer greater than or equal to 1.

24. An electronic device, comprising:

a processor; and

a memory, comprising one or more computer program modules,

wherein the one or more computer program modules are stored in the memory and configured to be executed by the processor, and the one or more computer program modules comprise instructions for performing the question recommendation method according to any one of claims 1 to 15.

25. A non-volatile readable storage medium, where computer instructions are stored, wherein the question recommendation method according to any one of claims 1 to 15 is executed in a case where the computer instructions are executed by a processor.

<u>10</u>

S100

Obtaining a candidate question set of a user, the candidate question set including a plurality of candidate questions

S140

Obtaining user behavior data, and obtaining a user interest parameter based on the user behavior data

S150

Based on the user interest parameter and the plurality of candidate questions, obtaining at least one similarity feature between each candidate question of the plurality of candidate questions and the user interest parameter

S160

Based on basic user information, the plurality of candidate questions, and the at least one similarity feature, sorting the plurality of candidate questions to obtain a question sequence

S170

Based on an order of the question sequence, recommending at least one candidate question in the question sequence to the user

FIG. 1A

10

Accessing a data knowledge base, the data knowledge base including a plurality of knowledge question sets

S110

Obtaining the basic user information, and establishing a user tag set based on the basic user information

S120

Associating the user tag set with the data knowledge base, and obtaining the candidate question set according to the plurality of knowledge question sets, the candidate question set including the plurality of candidate questions

S130

Obtaining user behavior data, and obtaining a user interest parameter based on the user behavior data

S140

Based on the user interest parameter and the plurality of candidate questions, obtaining at least one similarity feature between each candidate question of the plurality of candidate questions and the user interest parameter

S150

Based on basic user information, the plurality of candidate questions, and the at least one similarity feature, sorting the plurality of candidate questions to obtain a question sequence

S160

Based on an order of the question sequence, recommending at least one candidate question in the question sequence to the user

S170

FIG. 1B

Health Management Platform

Basic Information
Name          San Zhang
Gender             Male
Age                 68
Height          172cm
Weight          84kg
Waist Circumference     110


Which of the following lifestyle habits do you have?

☐ Regular drinking (more than three times a week)
☐ Smoking ☐   Salty diet
☐ Fond of fried foods
☐ Fond of sweets
☐ Often staying up late (average time to fall asleep later than 12 p.m.)
☐ Rarely exercise

FIG. 2A

User Portrait

Diabetic patient A

Height 172cm

Weight 81kg

Male

68 years old

Memory loss

Long-term leg ulcers

Thickened and swelled tiptoes

Big toe bone protruding

Alcohol and smoking often

Rarely exercise

Relevant Tags

"Blood Glucose"

"Old Age"

"Overweight"

"Exercise"

"Complication"

"Recipe"

"Neuropathy"

"Diabetic Foot"

FIG. 2B

Age:
Under 18 years old
18 to 59 years old
Over 60 years old

Type:
Type 1 Diabetes
Type 2 Diabetes
Elderly Diabetes
Gestational Diabetes

Time Period:
6:00-8:00    Diet, Blood Glucose
11:00-15:00. Diet, Exercise
17:00-20:00. Diet, Exercise,
             Blood Glucose,
             Medication

Complication

YES

NO

Type of
Complications:

Cardiovascular disease

Kidney disease

Retinopathy

Neuropathy

Lower extremity
vascular disease

Diabetic foot

YES

NO

Diabetes

Conditions:
Over 40 years old
Regularly sitting
Dyslipidemia

* * *

Fasting blood glucose

Blood glucose 2 hours
after meal

At least three
conditions &
6.1mmol/L<fasting blood
glucose<7mmol/L or
7.8mmol/L<blood glucose
2 hours after meal<
11.1mmol/L

Symptoms:

Hypertension

Difficulty in
urination

Memory loss

Night pain in
the lower limbs

* * *

FIG. 3A

Health Management Platform

How often do patients with hypertension monitor blood pressure every day?

The detection period should be determined by the condition. For patients with relatively stable conditions and little blood pressure fluctuations, blood pressure can be measured once a month. For patients whose blood pressure is difficult to stabilize and are in the stage of drug use adjustment, blood pressure should be measured once a week. In special circumstances, the measurement time should be determined in accordance with the doctor's advice. Thus, the harm of high blood pressure to the human body can be minimized.

Does the answer help you?

| YES | NO |

FIG. 3B

FIG. 4A

Health Management Platform

Can patients with hypertension have surgery?

What examination should patients with hypertension do?

What are the symptoms of patients with hypertension?

How often do patients with hypertension monitor blood pressure every day?

What exercise is suitable for patients with hypertension?

Guess you want to ask

Please enter the question you want to consult

Send

FIG. 4B

**Health Management Platform**

Guess you want to ask

Can patients with hypertension have surgery?

What examination should patients with hypertension do?

What are the symptoms of patients with hypertension?

How often do patients with hypertension monitor blood pressure every day?

What exercise is suitable for patients with hypertension?

| Hypertension diet | Precautions for hypertension diet | Gestational hypertension diet |
|---|---|---|

| Hypertension | Complete |
|---|---|

FIG. 4C

50

Establishing a data knowledge base — S510

Accessing a data knowledge base, the data knowledge base including a plurality of knowledge question sets — S520

Obtaining the basic user information, and establishing a user tag set based on the basic user information — S530

Associating the user tag set with the data knowledge base, and obtaining the candidate question set according to the plurality of knowledge question sets, the candidate question set including the plurality of candidate questions — S540

Obtaining user behavior data, and obtaining a user interest parameter based on the user behavior data — S550

Based on the user interest parameter and the plurality of candidate questions, obtaining at least one similarity feature between each candidate question of the plurality of candidate questions and the user interest parameter — S560

Based on basic user information, the plurality of candidate questions, and the at least one similarity feature, sorting the plurality of candidate questions to obtain a question sequence — S570

Based on an order of the question sequence, recommending at least one candidate question in the question sequence to the user — S580

FIG. 5A

FIG. 5B

FIG. 6

70

| Terminal 710 | ⟷ | Question Recommendation Server 720 | ⟷ | Knowledge Base Server 730 |

FIG. 7

80

Processor
810

820

Computer Program
Modules
821

FIG. 8

900

910 920 930

| Processing Device | ROM | RAM |

940

950

I/O Interface

| Input Device | Output Device | Storage Device | Communication Device |

960 970 980 990

FIG. 9

100

Computer Program
Instructions
111

FIG. 10

FIG. 11

<div style="text-align:center">

**INTERNATIONAL SEARCH REPORT**

</div>

| International application No. |
|---|
| **PCT/CN2020/093390** |

**A. CLASSIFICATION OF SUBJECT MATTER**

G06F 16/00(2019.01)i

According to International Patent Classification (IPC) or to both national classification and IPC

**B. FIELDS SEARCHED**

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNABS; CNTXT; VEN; USTXT; WOTXT; EPTXT; CNKI; IEEE: 问题, 画像, 知识图谱, 排序, 相似度, question, portrait, knowledge graph, rank, similarity

**C. DOCUMENTS CONSIDERED TO BE RELEVANT**

| Category* | Citation of document, with indication, where appropriate, of the relevant passages | Relevant to claim No. |
|---|---|---|
| X | CN 110377715 A (TIANJIN HUIZHIXINGYUAN INFORMATION TECHNOLOGY CO., LTD.) 25 October 2019 (2019-10-25) <br> description paragraphs 33-150 | 1-25 |
| X | CN 107451199 A (ALIBABA GROUP HOLDING LIMITED) 08 December 2017 (2017-12-08) <br> description paragraphs 64-172 | 1-25 |
| X | CN 105740476 A (KOORUN INTELLIGENCE TECHNOLOGY CO., LTD.) 06 July 2016 (2016-07-06) <br> description, paragraphs 48-92 | 1-25 |
| A | CN 110188186 A (PING AN TECHNOLOGY (SHENZHEN) CO., LTD.) 30 August 2019 (2019-08-30) <br> entire document | 1-25 |
| A | US 2020034465 A1 (INTERNATIONAL BUSINESS MACHINES CORPORATION) 30 January 2020 (2020-01-30) <br> entire document | 1-25 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 February 2021** | **09 March 2021** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**
Information on patent family members

| | | | International application No. |
|---|---|---|---|
| | | | **PCT/CN2020/093390** |

| Patent document cited in search report | | | Publication date (day/month/year) | Patent family member(s) | | | Publication date (day/month/year) |
|---|---|---|---|---|---|---|---|
| CN | 110377715 | A | 25 October 2019 | None | | | |
| CN | 107451199 | A | 08 December 2017 | CN | 107451199 | B | 26 June 2020 |
| CN | 105740476 | A | 06 July 2016 | None | | | |
| CN | 110188186 | A | 30 August 2019 | None | | | |
| US | 2020034465 | A1 | 30 January 2020 | None | | | |

Form PCT/ISA/210 (patent family annex) (January 2015)